# EUROPEAN PATENT SPECIFICATION

(11) **EP 0 977 875 B1**
(45) Date of publication and mention of the grant of the patent: **14.02.2007**
(21) Application number: 98917325.7
(22) Date of filing: 24.04.1998
(51) Int. Cl.: C12N 15/75, C12N 9/28, C12N 1/21, C12R 1/125, C12N 9/56

(54) **IMPROVED PROKARYOTIC EXPRESSION OF PROTEIN**
VERBESSERTE PROKARIONTISCHE PROTEINENEXPRESSION
EXPRESSION PROCARYOTIQUE AMELIOREE DE PROTEINES

(30) Priority: 26.04.1997 GB 9708452
(43) Date of publication of application: 09.02.2000
(73) Proprietor: NEWCASTLE UNIVERSITY VENTURES LIMITED, Newcastle upon Tyne NE1 1XX (GB); Novozymes A/S, 2880 Bagsvaerd (DK)
(72) Inventor: HARWOOD, Colin, Robert The University of Newcastle, Newcastle upon Tyne NE2 1HH (GB); STEPHENSON, Keith The University of Newcastle, Newcastle upon Tyne NE2 1HH (GB); JORGENSEN, Steen, DK-2880 Bagsvaerd (DK); JENSEN, Christina, DK-2880 Bagsvaerd (DK); KRISTENSEN, Tina, DK-2880 Bagsvaerd (DK)
(74) Representative: Kofoed, Gertrud Sonne
(86) International application number: PCT/GB1998/001051
(87) International publication number: WO 1998/049328

(56) References cited:
- EP-A- 0 369 817
- WO-A-92/16642
- MARGOT P ET AL: "The wprA gene of Bacillus subtilis 168, expressed during exponential growth, encodes a cell-wall-associated protease." MICROBIOLOGY (READING) 142 (12). 1996. 3437-3444. ISSN: 1350-0872, XP002075685
- SIMONEN M ET AL: "PROTEIN SECRETION IN BACILLUS SPECIES" MICROBIOLOGICAL REVIEWS, vol. 57, no. 1, 1 March 1993, pages 109-137, XP000562630

### SEQUENCE LISTING

(1) GENERAL INFORMATION:
   (i) APPLICANT:
      (A) NAME: UNIVERSITY OF NEWCASTLE UPON TYNE
      (B) STREET: 6 KENSINGTON TERRACE
      (C) CITY: NEWCASTLE UPON TYNE
      (D) STATE: TYNE & WEAR
      (E) COUNTRY: ENGLAND
      (F) POSTAL CODE (ZIP): NE1 7RU
      (G) TELEPHONE: ++44 191 222 5860
      (H) TELEFAX: ++44 191 222 5715
      (I) TELEX: none available

      (A) NAME: NOVO NORDISK A/S
      (B) STREET: NOVO ALLEE
      (C) CITY: BAGSVAERD
      (E) COUNTRY: DENMARK
      (F) POSTAL CODE (ZIP): 2880
      (G) TELEPHONE: ++45 4444 8888
      (H) TELEFAX: ++45 4449 0670
      (I) TELEX: none available
   (ii) TITLE OF INVENTION: IMPROVED PROKARYOTIC EXPRESSION OF PROTEIN
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: PCT/GB98/01051
   (vi) PRIOR APPLICATION DATA:
      (A) APPLICATION NUMBER: GB 9708452.9
      (B) FILING DATE: 26-APR-1997
(2) INFORMATION FOR SEQ ID NO: 1:
   (i) SEQUENCE CHARACTERISTICS:
      (A) LENGTH: 3117 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bacillus subtilis
      (B) STRAIN: DN1885
   (viii) POSITION IN GENOME:
      (C) UNITS: bp
   (ix) FEATURE:
      (A) NAME/KEY: CDS
      (B) LOCATION:154..2835
   (xi) SEQUENCE DESCRIPTION: SEQ ID NO: 1:
   AACCCGGCTC TTTGATAGAG CTGGTTTTTT TTATATTTAT CCCCTCATAT TCCAAATCAT      60
   TTAAAATAAC CTTAAAATTC CCTGTAAGCG GTATCTCGTC CTATGAAATT ATGATACCTT     120
   CAAGGAGATT CATTATTTTG CAGGAGGGAT AAC ATG AAA CGC AGA AAA TTC AGC     174
                                        Met Lys Arg Arg Lys Phe Ser
                                          1               5
   TCG GTT GTG GCG GCA GTG CTT ATT TTT GCA CTG ATT TTC AGC CTT TTT       222
   Ser Val Val Ala Ala Val Leu Ile Phe Ala Leu Ile Phe Ser Leu Phe
           10                  15                  20
   TCT CCG GGA ACC AAA GCT GCA GCG GCC GGC GCG ATC GAT CAG GCG GCT       270
   Ser Pro Gly Thr Lys Ala Ala Ala Ala Gly Ala Ile Asp Gln Ala Ala
       25                  30                  35
   GCT CTG GAA AAC GGC AAA GAG CAG ACA GGC GCC ATG AAG GAG CCG GAA       318
   Ala Leu Glu Asn Gly Lys Glu Gln Thr Gly Ala Met Lys Glu Pro Glu
    40                  45                  50                  55
   CAG GTG AAA TGG TAC AAA GTG ACC CCG GGA GCA ACG GAT ATT CAG AAA       366
   Gln Val Lys Trp Tyr Lys Val Thr Pro Gly Ala Thr Asp Ile Gln Lys
                   60                  65                  70
   AAC TCA CAT ATG GCA CTG ACC GTA AAG AGT GAT TCA GTA CTG AAT GTA       414
   Asn Ser His Met Ala Leu Thr Val Lys Ser Asp Ser Val Leu Asn Val
               75                  80                  85
   TCT GTA TAT CCA AGT AAG GAA AAA GCG CTT AAA GAT GAG ACG TTT GAG       462
   Ser Val Tyr Pro Ser Lys Glu Lys Ala Leu Lys Asp Glu Thr Phe Glu
           90                  95                 100
   ATG TAC CGT TCT TTC ACA GCG GAG GAT GGA AAA AGC GAA GTC ATT TTT       510
   Met Tyr Arg Ser Phe Thr Ala Glu Asp Gly Lys Ser Glu Val Ile Phe
       105                 110                 115
   CCA TAC GCG TGG AGC GGC CCT TAC TAT GTA AAA GTT GAA TAC CTC GGA       558
   Pro Tyr Ala Trp Ser Gly Pro Tyr Tyr Val Lys Val Glu Tyr Leu Gly
   120                 125                 130                 135
   GAA GAA GAA CCA GAG GAC GGC GGA ACG GCA GAA GCA GCT GCA GAA GCC       606
   Glu Glu Glu Pro Glu Asp Gly Gly Thr Ala Glu Ala Ala Ala Glu Ala
                   140                 145                 150
   AAG TAT ACG ATT GGG TAT AAA GGC ACC AAA AAA CAG CCG TCA GAT TTA       654
   Lys Tyr Thr Ile Gly Tyr Lys Gly Thr Lys Lys Gln Pro Ser Asp Leu
               155                 160                 165
   GAA GAG GAA GAA GCT TGT CCG GTT GAA ATG AGT GTC GAT CAG AAG AAA       702
   Glu Glu Glu Glu Ala Cys Pro Val Glu Met Ser Val Asp Gln Lys Lys
           170                 175                 180
   TCA GGA AAA GGC ATC CTG GAT AAG TTG AGA TCG ATT CGT GAT GAG CAG        750
   Ser Gly Lys Gly Ile Leu Asp Lys Leu Arg Ser Ile Arg Asp Glu Gln
       185                 190                 195
   CTG AGC CAA ACA GCA GAA GGC AAA GAA CTG ACA AGC CTT TAT TAC AAA        798
   Leu Ser Gln Thr Ala Glu Gly Lys Glu Leu Thr Ser Leu Tyr Tyr Lys
   200                 205                 210                 215
   GCA GCA CCG TTT ATC GTT GCA AAG CTC GCA CTC AAT AAA ACA GCA AGA        846
   Ala Ala Pro Phe Ile Val Ala Lys Leu Ala Leu Asn Lys Thr Ala Arg
                   220                 225                 230
   AAT GAA ATC TAT CAG GAT CTT GTG ACA TTA AAG CCG TTA TTT GAC GAT        894
   Asn Glu Ile Tyr Gln Asp Leu Val Thr Leu Lys Pro Leu Phe Asp Asp
               235                 240                 245
   GTG TCA GAA AAC GGA GCA TCA TCT TCG TAT AAG GTC ACT GAA AAG GAT        942
   Val Ser Glu Asn Gly Ala Ser Ser Ser Tyr Lys Val Thr Glu Lys Asp
           250                 255                 260
   CAA AAA GCG ATC AAC CGG CTA TAT GAT AAA GCT TTA CAA TCA GTC CCG        990
   Gln Lys Ala Ile Asn Arg Leu Tyr Asp Lys Ala Leu Gln Ser Val Pro
       265                 270                 275
   TCA TTC CTT AAA GAG GAG ATA AAG AAA CAA GCG GAC CGA CTA AAT ATG       1038
   Ser Phe Leu Lys Glu Glu Ile Lys Lys Gln Ala Asp Arg Leu Asn Met
   280                 285                 290                 295
   AAG CAG CTG CAA GGC AAA ACA GCA GGA GCC ATT TTA ACA GAA AAC AAT       1086
   Lys Gln Leu Gln Gly Lys Thr Ala Gly Ala Ile Leu Thr Glu Asn Asn
                   300                 305                 310
   ATT GCA GCA AAA AGT GAA GTT CAG ACA ACA AAG GTT ATT TTC AAG GTG       1134
   Ile Ala Ala Lys Ser Glu Val Gln Thr Thr Lys Val Ile Phe Lys Val
               315                 320                 325
   AAG GAC AAT AAA AGC CTC TCA TCC GTA CAT AAT GAA ATG AAG GGC TTT       1182
   Lys Asp Asn Lys Ser Leu Ser Ser Val His Asn Glu Met Lys Gly Phe
           330                 335                 340
   TCT GCA AGC GCG CAA TCG AAA AAA GAC ATA TCT AAT GTG AAA AAG GCA       1230
   Ser Ala Ser Ala Gln Ser Lys Lys Asp Ile Ser Asn Val Lys Lys Ala
       345                 350                 355
   AAG AAA CTG TTT GAC AAT CTG TAT TCA TTT GAA CTT CCG AAA GAC GAG       1278
   Lys Lys Leu Phe Asp Asn Leu Tyr Ser Phe Glu Leu Pro Lys Asp Glu
   360                 365                 370                 375
   AAA CAG AAC GGC GCA TAT ACG GCA AGC GCC AAA AGG GTC AAA AGC GCT       1326
   Lys Gln Asn Gly Ala Tyr Thr Ala Ser Ala Lys Arg Val Lys Ser Ala
                   380                 385                 390
   GCT GCG ACA CTA TCC AAG ATG TCC AAT GTA GAG TTT GCG GAA CCC GTA       1374
   Ala Ala Thr Leu Ser Lys Met Ser Asn Val Glu Phe Ala Glu Pro Val
               395                 400                 405
   CAG GAA TAC AAA AGC TTG GCA AAC GAT ATT CAG TAC CCT TAT CAA TGG       1422
   Gln Glu Tyr Lys Ser Leu Ala Asn Asp Ile Gln Tyr Pro Tyr Gln Trp
           410                 415                 420
   CCG CTT AAA AAC AAC GGT GAA AAC GGC GGT GTC AAA AAT GCG GAT GTG       1470
   Pro Leu Lys Asn Asn Gly Glu Asn Gly Gly Val Lys Asn Ala Asp Val
   425 430 435
   AAA TAT GAG CCT GCC AAC ACA TTG CTG TCC AAA CGC AAG CTT AAC GAT 1518
   Lys Tyr Glu Pro Ala Asn Thr Leu Leu Ser Lys Arg Lys Leu Asn Asp
   440 445 450 455
   ACA CTC ATT GCA GTA GTA GAC ACA GGC GTA GAC AGC ACG CTT GCC GAT 1566
   Thr Leu Ile Ala Val Val Asp Thr Gly Val Asp Ser Thr Leu Ala Asp
   460 465 470
   TTA AAA GGA AAA GTA AGA ACA GAT CTC GGA CAC AAT TTT GTC GGA CGA 1614
   Leu Lys Gly Lys Val Arg Thr Asp Leu Gly His Asn Phe Val Gly Arg
   475 480 485
   AAT AAC AAT GCA ATG GAT GAT CAG GGG CAT GGG ACG CAT GTC GCA GGC 1662
   Asn Asn Asn Ala Met Asp Asp Gln Gly His Gly Thr His Val Ala Gly
   490 495 500
   ATT ATT GCA GCC CAA AGC GAT AAC GGC TAT TCA ATG ACT GGA TTG AAT 1710
   Ile Ile Ala Ala Gln Ser Asp Asn Gly Tyr Ser Met Thr Gly Leu Asn
   505 510 515
   GCC AAA GCA AAA ATC ATC CCT GTA AAA GTG CTT GAT TCC GCA GGT TCC 1758
   Ala Lys Ala Lys Ile Ile Pro Val Lys Val Leu Asp Ser Ala Gly Ser
   520 525 530 535
   GGA GAT ACT GAA CAA ATT GCT CTC GGC ATC AAA TAT GCT GCT GAC AAA 1806
   Gly Asp Thr Glu Gln Ile Ala Leu Gly Ile Lys Tyr Ala Ala Asp Lys
   540 545 550
   GGA GCA AAG GTG ATT AAT TTA AGT TTA GGC GGA GGC TAC AGC CGC GTG 1854
   Gly Ala Lys Val Ile Asn Leu Ser Leu Gly Gly Gly Tyr Ser Arg Val
   555 560 565
   CTT GAA TTT GCT TTG AAG TAC GCA GCT GAC AAA AAT GTC TTG ATT GCC 1902
   Leu Glu Phe Ala Leu Lys Tyr Ala Ala Asp Lys Asn Val Leu Ile Ala
   570 575 580
   GCA GCC AGC GGG AAT GAT GGA GAA AAT GCC TTA TCT TAT CCT GCA TCT 1950
   Ala Ala Ser Gly Asn Asp Gly Glu Asn Ala Leu Ser Tyr Pro Ala Ser
   585 590 595
   TCT AAA TAT GTG ATG TCA GTC GGC GCA ACG AAT CGC ATG GAT ATG ACC 1998
   Ser Lys Tyr Val Met Ser Val Gly Ala Thr Asn Arg Met Asp Met Thr
   600 605 610 615
   GCT GAT TTC TCT AAC TAT GGA AAA GGT CTG GAC ATC TCT GCT CCA GGG 2046
   Ala Asp Phe Ser Asn Tyr Gly Lys Gly Leu Asp Ile Ser Ala Pro Gly
   620 625 630
   TCT GAT ATC CCG AGC TTA GTG CCG AAC GGA AAT GTC ACG TAC ATG AGC 2094
   Ser Asp Ile Pro Ser Leu Val Pro Asn Gly Asn Val Thr Tyr Met Ser
   635 640 645
   GGA ACG TCT ATG GCG ACG CCA TAT GCT GCC GCC GCT GCG GGA CTG CTG 2142
   Gly Thr Ser Met Ala Thr Pro Tyr Ala Ala Ala Ala Ala Gly Leu Leu
   650 655 660
   TTT GCT CAA AAT CCT AAG CTA AAA AGA ACA GAA GTT GAG GAT ATG TTG 2190
   Phe Ala Gln Asn Pro Lys Leu Lys Arg Thr Glu Val Glu Asp Met Leu
       665                 670                 675
   AAA AAG ACG GCA GAT GAT ATT TCC TTT GAA AGT GTC GAT GGC GGA GAA        2238
   Lys Lys Thr Ala Asp Asp Ile Ser Phe Glu Ser Val Asp Gly Gly Glu
   680                 685                 690                 695
   GAA GAG TTG TAT GAC GAT TAT GGC GAT CCG ATT GAA ATT CCG AAG ACA        2286
   Glu Glu Leu Tyr Asp Asp Tyr Gly Asp Pro Ile Glu Ile Pro Lys Thr
                   700                 705                 710
   CCT GGT GTA GAC TGG CAT TCA GGC TAC GGG CGG CTG AAT GTC ATG AAG        2334
   Pro Gly Val Asp Trp His Ser Gly Tyr Gly Arg Leu Asn Val Met Lys
               715                 720                 725
   GCT GTC AGC GCA GCT GAT TTA CAG CTT AAG GTC AAC AAG CTG GAA AGC        2382
   Ala Val Ser Ala Ala Asp Leu Gln Leu Lys Val Asn Lys Leu Glu Ser
           730                 735                 740
   ACT CAA ACA GCT GTC AGA GGA AGC GCG AAG GAA GGC ACC CTT ATC GAG        2430
   Thr Gln Thr Ala Val Arg Gly Ser Ala Lys Glu Gly Thr Leu Ile Glu
       745                 750                 755
   GTG ATG AAC GGC AAA AAG AAA CTC GGC AGC GCC AAA GCC GGA AAA GAC        2478
   Val Met Asn Gly Lys Lys Lys Leu Gly Ser Ala Lys Ala Gly Lys Asp
   760                 765                 770                 775
   AAT GCG TTC AAG GTG AAT ATC GCG ACT CAA AAA CAG GAT CAA GTA CTG        2526
   Asn Ala Phe Lys Val Asn Ile Ala Thr Gln Lys Gln Asp Gln Val Leu
                   780                 785                 790
   TAT CTG AAA GCA ACA AAA GGC GAT GCG AAA ACA TCG TAT AAA GTT GTT        2574
   Tyr Leu Lys Ala Thr Lys Gly Asp Ala Lys Thr Ser Tyr Lys Val Val
               795                 800                 805
   GTC GTC AAA GGA AAA CCT TCC GGC ACA CCG AAA GTA AAC GCG GTG AAA        2622
   Val Val Lys Gly Lys Pro Ser Gly Thr Pro Lys Val Asn Ala Val Lys
           810                 815                 820
   ACG AAG GAT ACG GCA GTA AAA GGG AAG GCA AAC AGC AAA GCG ATG ATC        2670
   Thr Lys Asp Thr Ala Val Lys Gly Lys Ala Asn Ser Lys Ala Met Ile
       825                 830                 835
   AGA GTG AAA AAC AAA TCA AAG AAA GTC ATT GCT TCT GCC AAA GCT GAC        2718
   Arg Val Lys Asn Lys Ser Lys Lys Val Ile Ala Ser Ala Lys Ala Asp
   840                 845                 850                 855
   GCA AAA GGA ACG TTT TCG GTG AAA ATC AAA AAA CAA AAA GCC GGA ACG        2766
   Ala Lys Gly Thr Phe Ser Val Lys Ile Lys Lys Gln Lys Ala Gly Thr
                   860                 865                 870
   GTG CTG TAC GTC ACG GCT GTG GAT ACA GAT AAA AAA GAA AGC AAG GAA        2814
   Val Leu Tyr Val Thr Ala Val Asp Thr Asp Lys Lys Glu Ser Lys Glu
               875                 880                 885
   GCA AAA GTT GTT GTT GAA AAG TAACCAAAAA GCGGTGCTCG ATGCACCGCT           2865
   Ala Lys Val Val Val Glu Lys
           890
   TTTTTATTTG CGCCCCCGTT GGACTGCTGT GTACATAATG GATCGCTTTC CGTTTGAAGC      2925
   CGTCCATTCA AAACGGTCTC CTGTCCCTTC GCTGCTGTAG TTGTAGTGAT GCTGCATCGC      2985
   GCATTGTGCG GTGTCATTTG AAAAGTGATA AAAGGACACA CCCTGCCCCG CCGAAGTATT 3045
   CGAAAAGACT CCTTTATTAT AAGAAAAGAC GCAATCGAAT ACCGATAAGC CGAAGCTGTG 3105
   CAAGTGCTGC AG 3117
(2) INFORMATION FOR SEQ ID NO: 2:
   (i) SEQUENCE CHARACTERISTICS:
      (A) LENGTH: 894 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Lys Arg Arg Lys Phe Ser Ser Val Val Ala Ala Val Leu Ile Phe
     1               5                  10                  15
   Ala Leu Ile Phe Ser Leu Phe Ser Pro Gly Thr Lys Ala Ala Ala Ala
                20                  25                  30
   Gly Ala Ile Asp Gln Ala Ala Ala Leu Glu Asn Gly Lys Glu Gln Thr
            35                  40                  45
   Gly Ala Met Lys Glu Pro Glu Gln Val Lys Trp Tyr Lys Val Thr Pro
        50                  55                  60
   Gly Ala Thr Asp Ile Gln Lys Asn Ser His Met Ala Leu Thr Val Lys
    65                  70                  75                  80
   Ser Asp Ser Val Leu Asn Val Ser Val Tyr Pro Ser Lys Glu Lys Ala
                    85                  90                  95
   Leu Lys Asp Glu Thr Phe Glu Met Tyr Arg Ser Phe Thr Ala Glu Asp
               100                 105                 110
   Gly Lys Ser Glu Val Ile Phe Pro Tyr Ala Trp Ser Gly Pro Tyr Tyr
           115                 120                 125
   Val Lys Val Glu Tyr Leu Gly Glu Glu Glu Pro Glu Asp Gly Gly Thr
       130                 135                 140
   Ala Glu Ala Ala Ala Glu Ala Lys Tyr Thr Ile Gly Tyr Lys Gly Thr
   145                 150                 155                 160
   Lys Lys Gln Pro Ser Asp Leu Glu Glu Glu Glu Ala Cys Pro Val Glu
                   165                 170                 175
   Met Ser Val Asp Gln Lys Lys Ser Gly Lys Gly Ile Leu Asp Lys Leu
               180                 185                 190
   Arg Ser Ile Arg Asp Glu Gln Leu Ser Gln Thr Ala Glu Gly Lys Glu
           195                 200                 205
   Leu Thr Ser Leu Tyr Tyr Lys Ala Ala Pro Phe Ile Val Ala Lys Leu
       210                 215                 220
   Ala Leu Asn Lys Thr Ala Arg Asn Glu Ile Tyr Gln Asp Leu Val Thr
   225 230 235 240
   Leu Lys Pro Leu Phe Asp Asp Val Ser Glu Asn Gly Ala Ser Ser Ser
   245 250 255
   Tyr Lys Val Thr Glu Lys Asp Gln Lys Ala Ile Asn Arg Leu Tyr Asp
   260 265 270
   Lys Ala Leu Gln Ser Val Pro Ser Phe Leu Lys Glu Glu Ile Lys Lys
   275 280 285
   Gln Ala Asp Arg Leu Asn Met Lys Gln Leu Gln Gly Lys Thr Ala Gly
   290 295 300
   Ala Ile Leu Thr Glu Asn Asn Ile Ala Ala Lys Ser Glu Val Gln Thr
   305 310 315 320
   Thr Lys Val Ile Phe Lys Val Lys Asp Asn Lys Ser Leu Ser Ser Val
   325 330 335
   His Asn Glu Met Lys Gly Phe Ser Ala Ser Ala Gln Ser Lys Lys Asp
   340 345 350
   Ile Ser Asn Val Lys Lys Ala Lys Lys Leu Phe Asp Asn Leu Tyr Ser
   355 360 365
   Phe Glu Leu Pro Lys Asp Glu Lys Gln Asn Gly Ala Tyr Thr Ala Ser
   370 375 380
   Ala Lys Arg Val Lys Ser Ala Ala Ala Thr Leu Ser Lys Met Ser Asn
   385 390 395 400
   Val Glu Phe Ala Glu Pro Val Gln Glu Tyr Lys Ser Leu Ala Asn Asp
   405 410 415
   Ile Gln Tyr Pro Tyr Gln Trp Pro Leu Lys Asn Asn Gly Glu Asn Gly
   420 425 430
   Gly Val Lys Asn Ala Asp Val Lys Tyr Glu Pro Ala Asn Thr Leu Leu
   435 440 445
   Ser Lys Arg Lys Leu Asn Asp Thr Leu Ile Ala Val Val Asp Thr Gly
   450 455 460
   Val Asp Ser Thr Leu Ala Asp Leu Lys Gly Lys Val Arg Thr Asp Leu
   465 470 475 480
   Gly His Asn Phe Val Gly Arg Asn Asn Asn Ala Met Asp Asp Gln Gly
   485 490 495
   His Gly Thr His Val Ala Gly Ile Ile Ala Ala Gln Ser Asp Asn Gly
   500 505 510
   Tyr Ser Met Thr Gly Leu Asn Ala Lys Ala Lys Ile Ile Pro Val Lys
   515 520 525
   Val Leu Asp Ser Ala Gly Ser Gly Asp Thr Glu Gln Ile Ala Leu Gly
   530 535 540
   Ile Lys Tyr Ala Ala Asp Lys Gly Ala Lys Val Ile Asn Leu Ser Leu
   545 550 555 560
   Gly Gly Gly Tyr Ser Arg Val Leu Glu Phe Ala Leu Lys Tyr Ala Ala
   565 570 575
   Asp Lys Asn Val Leu Ile Ala Ala Ala Ser Gly Asn Asp Gly Glu Asn
   580 585 590
   Ala Leu Ser Tyr Pro Ala Ser Ser Lys Tyr Val Met Ser Val Gly Ala
   595 600 605
   Thr Asn Arg Met Asp Met Thr Ala Asp Phe Ser Asn Tyr Gly Lys Gly
   610 615 620
   Leu Asp Ile Ser Ala Pro Gly Ser Asp Ile Pro Ser Leu Val Pro Asn
   625 630 635 640
   Gly Asn Val Thr Tyr Met Ser Gly Thr Ser Met Ala Thr Pro Tyr Ala
   645 650 655
   Ala Ala Ala Ala Gly Leu Leu Phe Ala Gln Asn Pro Lys Leu Lys Arg
   660 665 670
   Thr Glu Val Glu Asp Met Leu Lys Lys Thr Ala Asp Asp Ile Ser Phe
   675 680 685
   Glu Ser Val Asp Gly Gly Glu Glu Glu Leu Tyr Asp Asp Tyr Gly Asp
   690 695 700
   Pro Ile Glu Ile Pro Lys Thr Pro Gly Val Asp Trp His Ser Gly Tyr
   705 710 715 720
   Gly Arg Leu Asn Val Met Lys Ala Val Ser Ala Ala Asp Leu Gln Leu
   725 730 735
   Lys Val Asn Lys Leu Glu Ser Thr Gln Thr Ala Val Arg Gly Ser Ala
   740 745 750
   Lys Glu Gly Thr Leu Ile Glu Val Met Asn Gly Lys Lys Lys Leu Gly
   755 760 765
   Ser Ala Lys Ala Gly Lys Asp Asn Ala Phe Lys Val Asn Ile Ala Thr
   770 775 780
   Gln Lys Gln Asp Gln Val Leu Tyr Leu Lys Ala Thr Lys Gly Asp Ala
   785 790 795 800
   Lys Thr Ser Tyr Lys Val Val Val Val Lys Gly Lys Pro Ser Gly Thr
   805 810 815
   Pro Lys Val Asn Ala Val Lys Thr Lys Asp Thr Ala Val Lys Gly Lys
   820 825 830
   Ala Asn Ser Lys Ala Met Ile Arg Val Lys Asn Lys Ser Lys Lys Val
   835 840 845
   Ile Ala Ser Ala Lys Ala Asp Ala Lys Gly Thr Phe Ser Val Lys Ile
   850 855 860
   Lys Lys Gln Lys Ala Gly Thr Val Leu Tyr Val Thr Ala Val Asp Thr
   865 870 875 880
   Asp Lys Lys Glu Ser Lys Glu Ala Lys Val Val Val Glu Lys
   885 890

### SEQUENCE LISTING

(1) GENERAL INFORMATION:
   (i) APPLICANT:
      (A) NAME: UNIVERSITY OF NEWCASTLE UPON TYNE
      (B) STREET: 6 KENSINGTON TERRACE
      (C) CITY: NEWCASTLE UPON TYNE
      (D) STATE: TYNE & WEAR
      (E) COUNTRY: ENGLAND
      (F) POSTAL CODE (ZIP): NE1 7RU
      (G) TELEPHONE: ++44 191 222 5860
      (H) TELEFAX: ++44 191 222 5715
      (I) TELEX: none available

      (A) NAME: NOVO NORDISK A/S
      (B) STREET: NOVO ALLEE
      (C) CITY: BAGSVAERD
      (E) COUNTRY: DENMARK
      (F) POSTAL CODE (ZIP): 2880
      (G) TELEPHONE: ++45 4444 8888
      (H) TELEFAX: ++45 4449 0670
      (I) TELEX: none available
   (ii) TITLE OF INVENTION: IMPROVED PROKARYOTIC EXPRESSION OF PROTEIN
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
   (v) CURRENT APPLICATION DATA: APPLICATION NUMBER: PCT/GB98/01051
   (vi) PRIOR APPLICATION DATA:
      (A) APPLICATION NUMBER: GB 9708452.9
      (B) FILING DATE: 26-APR-1997
(2) INFORMATION FOR SEQ ID NO: 1:
   (i) SEQUENCE CHARACTERISTICS:
      (A) LENGTH: 3117 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bacillus subtilis
      (B) STRAIN: DN1885
   (viii) POSITION IN GENOME:
      (C) UNITS: bp
   (ix) FEATURE:
      (A) NAME/KEY: CDS
      (B) LOCATION:154..2835
   (xi) SEQUENCE DESCRIPTION: SEQ ID NO: 1:
   AACCCGGCTC TTTGATAGAG CTGGTTTTTT TTATATTTAT CCCCTCATAT TCCAAATCAT 60
   TTAAAATAAC CTTAAAATTC CCTGTAAGCG GTATCTCGTC CTATGAAATT ATGATACCTT 120
   CAAGGAGATT CATTATTTTG CAGGAGGGAT AAC ATG AAA CGC AGA AAA TTC AGC 174
   Met Lys Arg Arg Lys Phe Ser
   1 5
   TCG GTT GTG GCG GCA GTG CTT ATT TTT GCA CTG ATT TTC AGC CTT TTT 222
   Ser Val Val Ala Ala Val Leu Ile Phe Ala Leu Ile Phe Ser Leu Phe
   10 15 20
   TCT CCG GGA ACC AAA GCT GCA GCG GCC GGC GCG ATC GAT CAG GCG GCT 270
   Ser Pro Gly Thr Lys Ala Ala Ala Ala Gly Ala Ile Asp Gln Ala Ala
   25 30 35
   GCT CTG GAA AAC GGC AAA GAG CAG ACA GGC GCC ATG AAG GAG CCG GAA 318
   Ala Leu Glu Asn Gly Lys Glu Gln Thr Gly Ala Met Lys Glu Pro Glu
   40 45 50 55
   CAG GTG AAA TGG TAC AAA GTG ACC CCG GGA GCA ACG GAT ATT CAG AAA 366
   Gln Val Lys Trp Tyr Lys Val Thr Pro Gly Ala Thr Asp Ile Gln Lys
   60 65 70
   AAC TCA CAT ATG GCA CTG ACC GTA AAG AGT GAT TCA GTA CTG AAT GTA 414
   Asn Ser His Met Ala Leu Thr Val Lys Ser Asp Ser Val Leu Asn Val
   75 80 85
   TCT GTA TAT CCA AGT AAG GAA AAA GCG CTT AAA GAT GAG ACG TTT GAG 462
   Ser Val Tyr Pro Ser Lys Glu Lys Ala Leu Lys Asp Glu Thr Phe Glu
   90 95 100
   ATG TAC CGT TCT TTC ACA GCG GAG GAT GGA AAA AGC GAA GTC ATT TTT 510
   Met Tyr Arg Ser Phe Thr Ala Glu Asp Gly Lys Ser Glu Val Ile Phe
   105 110 115
   CCA TAC GCG TGG AGC GGC CCT TAC TAT GTA AAA GTT GAA TAC CTC GGA 558
   Pro Tyr Ala Trp Ser Gly Pro Tyr Tyr Val Lys Val Glu Tyr Leu Gly
   120 125 130 135
   GAA GAA GAA CCA GAG GAC GGC GGA ACG GCA GAA GCA GCT GCA GAA GCC 606
   Glu Glu Glu Pro Glu Asp Gly Gly Thr Ala Glu Ala Ala Ala Glu Ala
   140 145 150
   AAG TAT ACG ATT GGG TAT AAA GGC ACC AAA AAA CAG CCG TCA GAT TTA 654
   Lys Tyr Thr Ile Gly Tyr Lys Gly Thr Lys Lys Gln Pro Ser Asp Leu
   155 160 165
   GAA GAG GAA GAA GCT TGT CCG GTT GAA ATG AGT GTC GAT CAG AAG AAA 702
   Glu Glu Glu Glu Ala Cys Pro Val Glu Met Ser Val Asp Gln Lys Lys
   170 175 180
   TCA GGA AAA GGC ATC CTG GAT AAG TTG AGA TCG ATT CGT GAT GAG CAG      750
   Ser Gly Lys Gly Ile Leu Asp Lys Leu Arg Ser Ile Arg Asp Glu Gln
       185                 190                 195
   CTG AGC CAA ACA GCA GAA GGC AAA GAA CTG ACA AGC CTT TAT TAC AAA      798
   Leu Ser Gln Thr Ala Glu Gly Lys Glu Leu Thr Ser Leu Tyr Tyr Lys
   200                 205                 210                 215
   GCA GCA CCG TTT ATC GTT GCA AAG CTC GCA CTC AAT AAA ACA GCA AGA      846
   Ala Ala Pro Phe Ile Val Ala Lys Leu Ala Leu Asn Lys Thr Ala Arg
                   220                 225                 230
   AAT GAA ATC TAT CAG GAT CTT GTG ACA TTA AAG CCG TTA TTT GAC GAT      894
   Asn Glu Ile Tyr Gln Asp Leu Val Thr Leu Lys Pro Leu Phe Asp Asp
               235                 240                 245
   GTG TCA GAA AAC GGA GCA TCA TCT TCG TAT AAG GTC ACT GAA AAG GAT      942
   Val Ser Glu Asn Gly Ala Ser Ser Ser Tyr Lys Val Thr Glu Lys Asp
           250                 255                 260
   CAA AAA GCG ATC AAC CGG CTA TAT GAT AAA GCT TTA CAA TCA GTC CCG      990
   Gln Lys Ala Ile Asn Arg Leu Tyr Asp Lys Ala Leu Gln Ser Val Pro
       265                 270                 275
   TCA TTC CTT AAA GAG GAG ATA AAG AAA CAA GCG GAC CGA CTA AAT ATG     1038
   Ser Phe Leu Lys Glu Glu Ile Lys Lys Gln Ala Asp Arg Leu Asn Met
   280                 285                 290                 295
   AAG CAG CTG CAA GGC AAA ACA GCA GGA GCC ATT TTA ACA GAA AAC AAT     1086
   Lys Gln Leu Gln Gly Lys Thr Ala Gly Ala Ile Leu Thr Glu Asn Asn
                   300                 305                 310
   ATT GCA GCA AAA AGT GAA GTT CAG ACA ACA AAG GTT ATT TTC AAG GTG     1134
   Ile Ala Ala Lys Ser Glu Val Gln Thr Thr Lys Val Ile Phe Lys Val
               315                 320                 325
   AAG GAC AAT AAA AGC CTC TCA TCC GTA CAT AAT GAA ATG AAG GGC TTT     1182
   Lys Asp Asn Lys Ser Leu Ser Ser Val His Asn Glu Met Lys Gly Phe
           330                 335                 340
   TCT GCA AGC GCG CAA TCG AAA AAA GAC ATA TCT AAT GTG AAA AAG GCA     1230
   Ser Ala Ser Ala Gln Ser Lys Lys Asp Ile Ser Asn Val Lys Lys Ala
       345                 350                 355
   AAG AAA CTG TTT GAC AAT CTG TAT TCA TTT GAA CTT CCG AAA GAC GAG     1278
   Lys Lys Leu Phe Asp Asn Leu Tyr Ser Phe Glu Leu Pro Lys Asp Glu
   360                 365                 370                 375
   AAA CAG AAC GGC GCA TAT ACG GCA AGC GCC AAA AGG GTC AAA AGC GCT     1326
   Lys Gln Asn Gly Ala Tyr Thr Ala Ser Ala Lys Arg Val Lys Ser Ala
                   380                 385                 390
   GCT GCG ACA CTA TCC AAG ATG TCC AAT GTA GAG TTT GCG GAA CCC GTA     1374
   Ala Ala Thr Leu Ser Lys Met Ser Asn Val Glu Phe Ala Glu Pro Val
               395                 400                 405
   CAG GAA TAC AAA AGC TTG GCA AAC GAT ATT CAG TAC CCT TAT CAA TGG     1422
   Gln Glu Tyr Lys Ser Leu Ala Asn Asp Ile Gln Tyr Pro Tyr Gln Trp
           410                 415                 420
   CCG CTT AAA AAC AAC GGT GAA AAC GGC GGT GTC AAA AAT GCG GAT GTG     1470
   Pro Leu Lys Asn Asn Gly Glu Asn Gly Gly Val Lys Asn Ala Asp Val
       425                 430                 435
   AAA TAT GAG CCT GCC AAC ACA TTG CTG TCC AAA CGC AAG CTT AAC GAT        1518
   Lys Tyr Glu Pro Ala Asn Thr Leu Leu Ser Lys Arg Lys Leu Asn Asp
   440                 445                 450                 455
   ACA CTC ATT GCA GTA GTA GAC ACA GGC GTA GAC AGC ACG CTT GCC GAT        1566
   Thr Leu Ile Ala Val Val Asp Thr Gly Val Asp Ser Thr Leu Ala Asp
                   460                 465                 470
   TTA AAA GGA AAA GTA AGA ACA GAT CTC GGA CAC AAT TTT GTC GGA CGA        1614
   Leu Lys Gly Lys Val Arg Thr Asp Leu Gly His Asn Phe Val Gly Arg
               475                 480                 485
   AAT AAC AAT GCA ATG GAT GAT CAG GGG CAT GGG ACG CAT GTC GCA GGC        1662
   Asn Asn Asn Ala Met Asp Asp Gln Gly His Gly Thr His Val Ala Gly
           490                 495                 500
   ATT ATT GCA GCC CAA AGC GAT AAC GGC TAT TCA ATG ACT GGA TTG AAT        1710
   Ile Ile Ala Ala Gln Ser Asp Asn Gly Tyr Ser Met Thr Gly Leu Asn
       505                 510                 515
   GCC AAA GCA AAA ATC ATC CCT GTA AAA GTG CTT GAT TCC GCA GGT TCC        1758
   Ala Lys Ala Lys Ile Ile Pro Val Lys Val Leu Asp Ser Ala Gly Ser
   520                 525                 530                 535
   GGA GAT ACT GAA CAA ATT GCT CTC GGC ATC AAA TAT GCT GCT GAC AAA        1806
   Gly Asp Thr Glu Gln Ile Ala Leu Gly Ile Lys Tyr Ala Ala Asp Lys
                   540                 545                 550
   GGA GCA AAG GTG ATT AAT TTA AGT TTA GGC GGA GGC TAC AGC CGC GTG        1854
   Gly Ala Lys Val Ile Asn Leu Ser Leu Gly Gly Gly Tyr Ser Arg Val
               555                 560                 565
   CTT GAA TTT GCT TTG AAG TAC GCA GCT GAC AAA AAT GTC TTG ATT GCC        1902
   Leu Glu Phe Ala Leu Lys Tyr Ala Ala Asp Lys Asn Val Leu Ile Ala
           570                 575                 580
   GCA GCC AGC GGG AAT GAT GGA GAA AAT GCC TTA TCT TAT CCT GCA TCT        1950
   Ala Ala Ser Gly Asn Asp Gly Glu Asn Ala Leu Ser Tyr Pro Ala Ser
       585                 590                 595
   TCT AAA TAT GTG ATG TCA GTC GGC GCA ACG AAT CGC ATG GAT ATG ACC        1998
   Ser Lys Tyr Val Met Ser Val Gly Ala Thr Asn Arg Met Asp Met Thr
   600                 605                 610                 615
   GCT GAT TTC TCT AAC TAT GGA AAA GGT CTG GAC ATC TCT GCT CCA GGG        2046
   Ala Asp Phe Ser Asn Tyr Gly Lys Gly Leu Asp Ile Ser Ala Pro Gly
                   620                 625                 630
   TCT GAT ATC CCG AGC TTA GTG CCG AAC GGA AAT GTC ACG TAC ATG AGC        2094
   Ser Asp Ile Pro Ser Leu Val Pro Asn Gly Asn Val Thr Tyr Met Ser
               635                 640                 645
   GGA ACG TCT ATG GCG ACG CCA TAT GCT GCC GCC GCT GCG GGA CTG CTG        2142
   Gly Thr Ser Met Ala Thr Pro Tyr Ala Ala Ala Ala Ala Gly Leu Leu
           650                 655                 660
   TTT GCT CAA AAT CCT AAG CTA AAA AGA ACA GAA GTT GAG GAT ATG TTG        2190
   Phe Ala Gln Asn Pro Lys Leu Lys Arg Thr Glu Val Glu Asp Met Leu
           665                 670                 675
   AAA AAG ACG GCA GAT GAT ATT TCC TTT GAA AGT GTC GAT GGC GGA GAA       2238
   Lys Lys Thr Ala Asp Asp Ile Ser Phe Glu Ser Val Asp Gly Gly Glu
   680                 685                 690                 695
   GAA GAG TTG TAT GAC GAT TAT GGC GAT CCG ATT GAA ATT CCG AAG ACA       2286
   Glu Glu Leu Tyr Asp Asp Tyr Gly Asp Pro Ile Glu Ile Pro Lys Thr
                   700                 705                 710
   CCT GGT GTA GAC TGG CAT TCA GGC TAC GGG CGG CTG AAT GTC ATG AAG       2334
   Pro Gly Val Asp Trp His Ser Gly Tyr Gly Arg Leu Asn Val Met Lys
               715                 720                 725
   GCT GTC AGC GCA GCT GAT TTA CAG CTT AAG GTC AAC AAG CTG GAA AGC       2382
   Ala Val Ser Ala Ala Asp Leu Gln Leu Lys Val Asn Lys Leu Glu Ser
           730                 735                 740
   ACT CAA ACA GCT GTC AGA GGA AGC GCG AAG GAA GGC ACC CTT ATC GAG       2430
   Thr Gln Thr Ala Val Arg Gly Ser Ala Lys Glu Gly Thr Leu Ile Glu
       745                 750                 755
   GTG ATG AAC GGC AAA AAG AAA CTC GGC AGC GCC AAA GCC GGA AAA GAC       2478
   Val Met Asn Gly Lys Lys Lys Leu Gly Ser Ala Lys Ala Gly Lys Asp
   760                 765                 770                 775
   AAT GCG TTC AAG GTG AAT ATC GCG ACT CAA AAA CAG GAT CAA GTA CTG       2526
   Asn Ala Phe Lys Val Asn Ile Ala Thr Gln Lys Gln Asp Gln Val Leu
                   780                 785                 790
   TAT CTG AAA GCA ACA AAA GGC GAT GCG AAA ACA TCG TAT AAA GTT GTT       2574
   Tyr Leu Lys Ala Thr Lys Gly Asp Ala Lys Thr Ser Tyr Lys Val Val
               795                 800                 805
   GTC GTC AAA GGA AAA CCT TCC GGC ACA CCG AAA GTA AAC GCG GTG AAA       2622
   Val Val Lys Gly Lys Pro Ser Gly Thr Pro Lys Val Asn Ala Val Lys
           810                 815                 820
   ACG AAG GAT ACG GCA GTA AAA GGG AAG GCA AAC AGC AAA GCG ATG ATC       2670
   Thr Lys Asp Thr Ala Val Lys Gly Lys Ala Asn Ser Lys Ala Met Ile
       825                 830                 835
   AGA GTG AAA AAC AAA TCA AAG AAA GTC ATT GCT TCT GCC AAA GCT GAC       2718
   Arg Val Lys Asn Lys Ser Lys Lys Val Ile Ala Ser Ala Lys Ala Asp
   840                 845                 850                 855
   GCA AAA GGA ACG TTT TCG GTG AAA ATC AAA AAA CAA AAA GCC GGA ACG       2766
   Ala Lys Gly Thr Phe Ser Val Lys Ile Lys Lys Gln Lys Ala Gly Thr
                   860                 865                 870
   GTG CTG TAC GTC ACG GCT GTG GAT ACA GAT AAA AAA GAA AGC AAG GAA       2814
   Val Leu Tyr Val Thr Ala Val Asp Thr Asp Lys Lys Glu Ser Lys Glu
               875                 880                 885
   GCA AAA GTT GTT GTT GAA AAG TAACCAAAAA GCGGTGCTCG ATGCACCGCT          2865
   Ala Lys Val Val Val Glu Lys
           890
   TTTTTATTTG CGCCCCCGTT GGACTGCTGT GTACATAATG GATCGCTTTC CGTTTGAAGC     2925
   CGTCCATTCA AAACGGTCTC CTGTCCCTTC GCTGCTGTAG TTGTAGTGAT GCTGCATCGC     2985
   GCATTGTGCG GTGTCATTTG AAAAGTGATA AAAGGACACA CCCTGCCCCG CCGAAGTATT 3045
   CGAAAAGACT CCTTTATTAT AAGAAAAGAC GCAATCGAAT ACCGATAAGC CGAAGCTGTG 3105
   CAAGTGCTGC AG 3117
(2) INFORMATION FOR SEQ ID NO: 2:
   (i) SEQUENCE CHARACTERISTICS:
      (A) LENGTH: 894 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Lys Arg Arg Lys Phe Ser Ser Val Val Ala Ala Val Leu Ile Phe
    1               5                  10                  15
   Ala Leu Ile Phe Ser Leu Phe Ser Pro Gly Thr Lys Ala Ala Ala Ala
               20                  25                  30
   Gly Ala Ile Asp Gln Ala Ala Ala Leu Glu Asn Gly Lys Glu Gln Thr
           35                  40                  45
   Gly Ala Met Lys Glu Pro Glu Gln Val Lys Trp Tyr Lys Val Thr Pro
       50                  55                  60
   Gly Ala Thr Asp Ile Gln Lys Asn Ser His Met Ala Leu Thr Val Lys
   65                  70                  75                  80
   Ser Asp Ser Val Leu Asn Val Ser Val Tyr Pro Ser Lys Glu Lys Ala
                   85                  90                  95
   Leu Lys Asp Glu Thr Phe Glu Met Tyr Arg Ser Phe Thr Ala Glu Asp
               100                 105                 110
   Gly Lys Ser Glu Val Ile Phe Pro Tyr Ala Trp Ser Gly Pro Tyr Tyr
           115                 120                 125
   Val Lys Val Glu Tyr Leu Gly Glu Glu Glu Pro Glu Asp Gly Gly Thr
       130                 135                 140
   Ala Glu Ala Ala Ala Glu Ala Lys Tyr Thr Ile Gly Tyr Lys Gly Thr
   145                 150                 155                 160
   Lys Lys Gln Pro Ser Asp Leu Glu Glu Glu Glu Ala Cys Pro Val Glu
                   165                 170                 175
   Met Ser Val Asp Gln Lys Lys Ser Gly Lys Gly Ile Leu Asp Lys Leu
               180                 185                 190
   Arg Ser Ile Arg Asp Glu Gln Leu Ser Gln Thr Ala Glu Gly Lys Glu
           195                 200                 205
   Leu Thr Ser Leu Tyr Tyr Lys Ala Ala Pro Phe Ile Val Ala Lys Leu
       210                 215                 220
   Ala Leu Asn Lys Thr Ala Arg Asn Glu Ile Tyr Gln Asp Leu Val Thr
   225                 230                 235                 240
   Leu Lys Pro Leu Phe Asp Asp Val Ser Glu Asn Gly Ala Ser Ser Ser
                   245                 250                 255
   Tyr Lys Val Thr Glu Lys Asp Gln Lys Ala Ile Asn Arg Leu Tyr Asp
               260                 265                 270
   Lys Ala Leu Gln Ser Val Pro Ser Phe Leu Lys Glu Glu Ile Lys Lys
           275                 280                 285
   Gln Ala Asp Arg Leu Asn Met Lys Gln Leu Gln Gly Lys Thr Ala Gly
       290                 295                 300
   Ala Ile Leu Thr Glu Asn Asn Ile Ala Ala Lys Ser Glu Val Gln Thr
   305                 310                 315                 320
   Thr Lys Val Ile Phe Lys Val Lys Asp Asn Lys Ser Leu Ser Ser Val
                   325                 330                 335
   His Asn Glu Met Lys Gly Phe Ser Ala Ser Ala Gln Ser Lys Lys Asp
               340                 345                 350
   Ile Ser Asn Val Lys Lys Ala Lys Lys Leu Phe Asp Asn Leu Tyr Ser
           355                 360                 365
   Phe Glu Leu Pro Lys Asp Glu Lys Gln Asn Gly Ala Tyr Thr Ala Ser
       370                 375                 380
   Ala Lys Arg Val Lys Ser Ala Ala Ala Thr Leu Ser Lys Met Ser Asn
   385                 390                 395                 400
   Val Glu Phe Ala Glu Pro Val Gln Glu Tyr Lys Ser Leu Ala Asn Asp
                   405                 410                 415
   Ile Gln Tyr Pro Tyr Gln Trp Pro Leu Lys Asn Asn Gly Glu Asn Gly
               420                 425                 430
   Gly Val Lys Asn Ala Asp Val Lys Tyr Glu Pro Ala Asn Thr Leu Leu
           435                 440                 445
   Ser Lys Arg Lys Leu Asn Asp Thr Leu Ile Ala Val Val Asp Thr Gly
       450                 455                 460
   Val Asp Ser Thr Leu Ala Asp Leu Lys Gly Lys Val Arg Thr Asp Leu
   465                 470                 475                 480
   Gly His Asn Phe Val Gly Arg Asn Asn Asn Ala Met Asp Asp Gln Gly
                   485                 490                 495
   His Gly Thr His Val Ala Gly Ile Ile Ala Ala Gln Ser Asp Asn Gly
               500                 505                 510
   Tyr Ser Met Thr Gly Leu Asn Ala Lys Ala Lys Ile Ile Pro Val Lys
           515                 520                 525
   Val Leu Asp Ser Ala Gly Ser Gly Asp Thr Glu Gln Ile Ala Leu Gly
       530                 535                 540
   Ile Lys Tyr Ala Ala Asp Lys Gly Ala Lys Val Ile Asn Leu Ser Leu
   545                 550                 555                 560
   Gly Gly Gly Tyr Ser Arg Val Leu Glu Phe Ala Leu Lys Tyr Ala Ala
                   565                 570                 575
   Asp Lys Asn Val Leu Ile Ala Ala Ala Ser Gly Asn Asp Gly Glu Asn
               580                 585                 590
   Ala Leu Ser Tyr Pro Ala Ser Ser Lys Tyr Val Met Ser Val Gly Ala
           595                 600                 605
   Thr Asn Arg Met Asp Met Thr Ala Asp Phe Ser Asn Tyr Gly Lys Gly
       610                 615                 620
   Leu Asp Ile Ser Ala Pro Gly Ser Asp Ile Pro Ser Leu Val Pro Asn
   625                 630                 635                 640
   Gly Asn Val Thr Tyr Met Ser Gly Thr Ser Met Ala Thr Pro Tyr Ala
                   645                 650                 655
   Ala Ala Ala Ala Gly Leu Leu Phe Ala Gln Asn Pro Lys Leu Lys Arg
               660                 665                 670
   Thr Glu Val Glu Asp Met Leu Lys Lys Thr Ala Asp Asp Ile Ser Phe
           675                 680                 685
   Glu Ser Val Asp Gly Gly Glu Glu Glu Leu Tyr Asp Asp Tyr Gly Asp
       690                 695                 700
   Pro Ile Glu Ile Pro Lys Thr Pro Gly Val Asp Trp His Ser Gly Tyr
   705                 710                 715                 720
   Gly Arg Leu Asn Val Met Lys Ala Val Ser Ala Ala Asp Leu Gln Leu
                   725                 730                 735
   Lys Val Asn Lys Leu Glu Ser Thr Gln Thr Ala Val Arg Gly Ser Ala
               740                 745                 750
   Lys Glu Gly Thr Leu Ile Glu Val Met Asn Gly Lys Lys Lys Leu Gly
           755                 760                 765
   Ser Ala Lys Ala Gly Lys Asp Asn Ala Phe Lys Val Asn Ile Ala Thr
       770                 775                 780
   Gln Lys Gln Asp Gln Val Leu Tyr Leu Lys Ala Thr Lys Gly Asp Ala
   785                 790                 795                 800
   Lys Thr Ser Tyr Lys Val Val Val Val Lys Gly Lys Pro Ser Gly Thr
                   805                 810                 815
   Pro Lys Val Asn Ala Val Lys Thr Lys Asp Thr Ala Val Lys Gly Lys
               820                 825                 830
   Ala Asn Ser Lys Ala Met Ile Arg Val Lys Asn Lys Ser Lys Lys Val
           835                 840                 845
   Ile Ala Ser Ala Lys Ala Asp Ala Lys Gly Thr Phe Ser Val Lys Ile
       850                 855                 860
   Lys Lys Gln Lys Ala Gly Thr Val Leu Tyr Val Thr Ala Val Asp Thr
   865                 870                 875                 880
   Asp Lys Lys Glu Ser Lys Glu Ala Lys Val Val Val Glu Lys
                   885                 890

## Description

The invention relates to a novel prokaryotic expression system and proteins expressed thereby.

The industrial production of proteins has, in many instances, exploited the native expression and secretory systems of micro-organisms and specifically bacteria. For example, and without limitation, the bacterium *Bacillus subtilis* is known to produce and secrete a number of proteins. One of these proteins α-amylase is of industrial importance and therefore the harvesting of this secreted protein is an activity currently undertaken by industry. However, the yield of this protein is significantly reduced by protein degradation during or immediately after, or just after, passage through the cell membrane.

It therefore follows that there is a need to provide a protein expression system which enhances the production of native protein or indeed heterologous or recombinant protein and more specifically enhances this production by reducing the degradation of protein.

It is also known to provide microorganisms that express and, advantageously, secrete heterologous protein i.e. proteins that are not native to that particular bacteria. This sort of system typically involves transformation of a bacterial cell with heterologous DNA with a view to manufacturing or producing recombinant protein. Microorganisms such as *Escherichia coli* (bacteria) *Saccharomyces cerevisiae, Aspergillus nidulans* and *Neurospora crassa* (fungi) have been used in this fashion.

The expression of heterologous protein in these primitive eukaryotes also allows some desirable eukaryotic post translational modifications to occur in these heterologous proteins leading to increases in the stability of the expressed protein and subsequent improvements in yield. More recently the use of mammalian and insect cell culture systems have been developed to facilitate the expression of eukaryotic proteins that for various reasons cannot be expressed in a prokaryotic host cell.

However the cost effectiveness of producing recombinant protein still remains the major advantage offered by genetically engineered prokaryotic expression systems and indeed significant advances have been made in the development of genetically engineered *E.coli* strains that increase the yield of specific recombinant proteins. The evolution of these bacterial strains has also been married with an ever increasing development of more efficient vectors adapted to optimize the expression of recombinant protein. These vectors commonly contain promoter elements that can be switched on or off with ease.

However there are two major disadvantages when using *E.coli* as a means of expressing recombinant protein. Firstly the high levels of expression may lead to a precipitation of recombinant protein in the bacterial cytoplasm as "inclusion bodies". This feature was thought to be advantageous as it can provide a simple means of separating the insoluble recombinant protein from the soluble endogenous *E.coli* protein. However, in reality this advantage is not a general feature of the system as in many cases the protein remains as an insoluble precipitate that can only be released into solution by using strong chaotropic agents. This presents a major problem if the protein in question is particularly labile and therefore loses biochemical or biological activity upon denaturation. Secondly the expression of foreign protein in *E.coli* leads to the rapid degradation of these proteins via an efficient proteolytic system. Therefore difficulties arise with regard to the isolation of intact recombinant protein from *E.coli* cells.

*E. coli* strains (TOPP series, BL21) have been engineered to allow the expression of recombinant proteins that would ordinarily be difficult to express in traditional laboratory strains of *E. coli.* However these engineered. *E.coli* strains are invariably not as biologically disabled as traditional laboratory strains of *E. coli* and as a consequence require containment levels that are higher than what would normally be required.

The identification of alternative prokaryotic host cells and the development of means that facilitate the production of soluble, intact and biologically active protein is obviously desirable. However, notably the number of potential prokaryotic host cells is huge.

With a view to producing a novel protein expression system we have chosen to genetically engineer, as our example, *Bacillus subtilis* in order to provide an expression system that overcomes the problems of yield associated with prior art systems. We have focused our attention on providing a bacterial expression system that produces and ideally secretes proteins (native and/or heterologous and/or recombinant) into the culture medium because this system enables purification of the manufactured protein due to the absence of contaminating endogenous bacterial proteins and other macromolecules.

A number of *B. subtilis* genes encoding secreted proteases have been identified. For example, without limitation, *aprE, nprE, bpf, mpr, epr, nprB* and *vpr* genes of B. subtilis encode extracellular proteases. These proteases are secreted into the culture medium and deletion of them from the *B. subtilis* genome reduces extracellular protease activity to less than 1% of wild type strains. Despite this fact experimental evidence suggests that *B. subtilis* strains deficient for extracellular proteases still show significant loss of secretory protein production through proteolytic degradation.

The identification of a cell wall-associated protease has lead us to investigate whether it has a role in expression and secretion of native and/or heterologous and/or recombinant protein in *B. subtilis* and whether or not the protease plays a major role in determining the levels of secretory protein production.

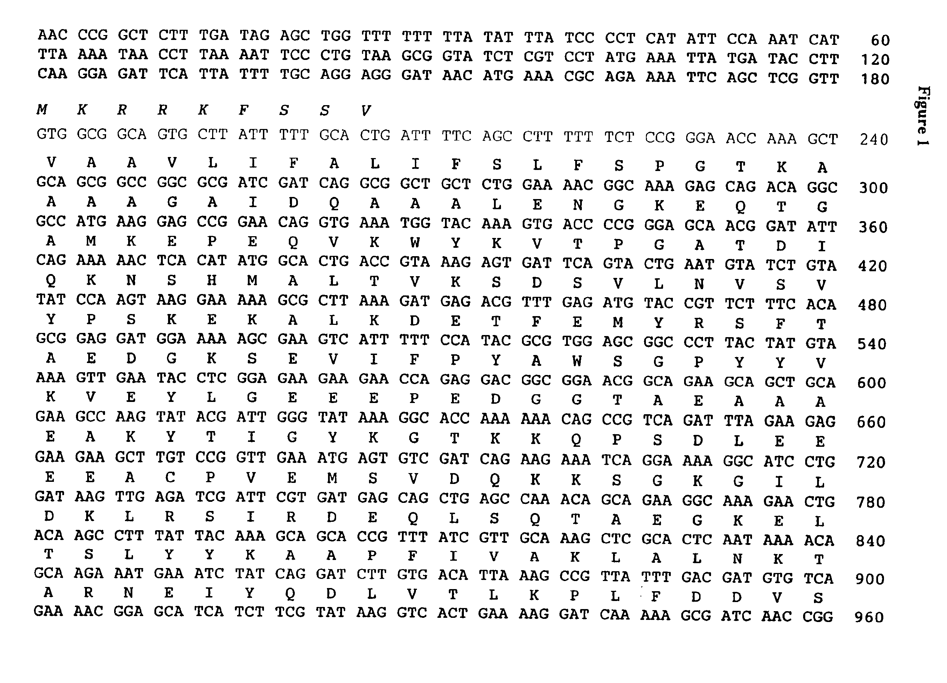

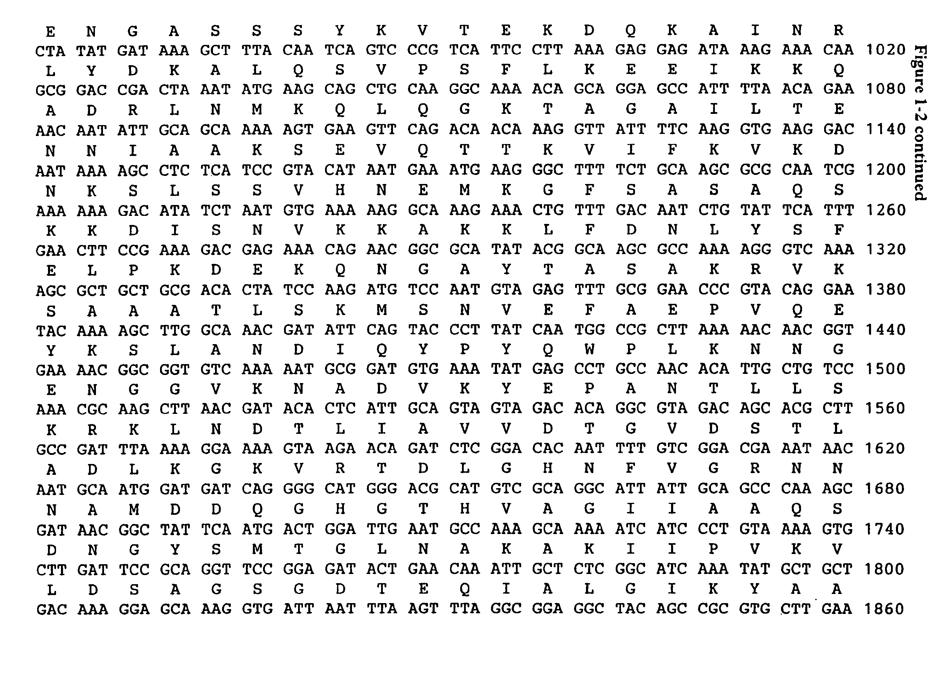

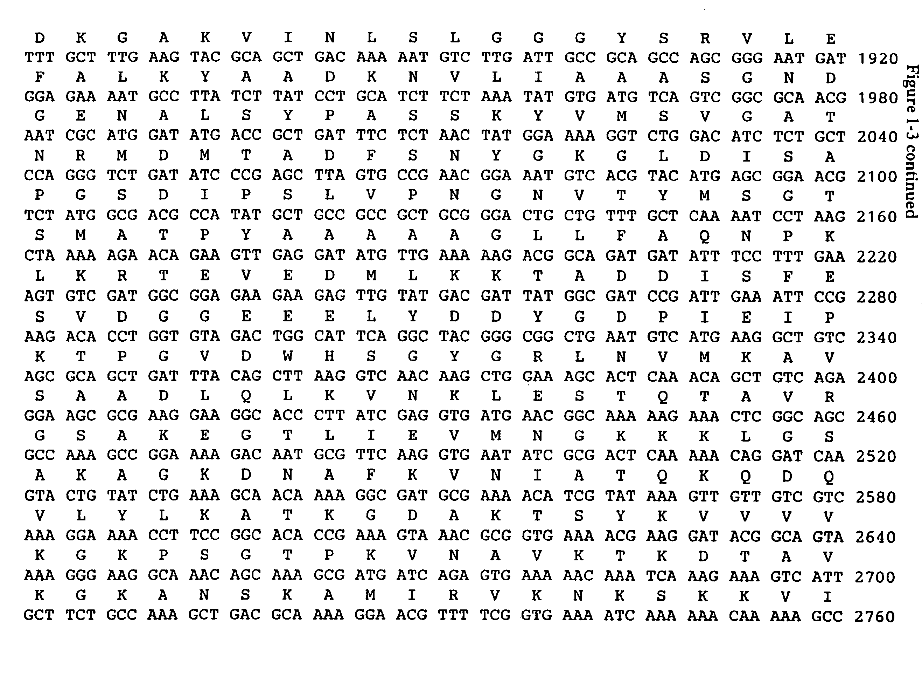

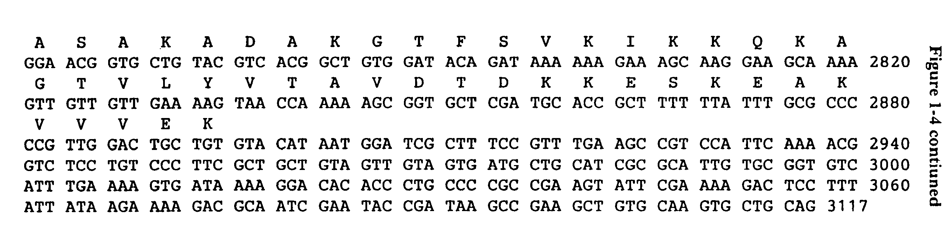

The wall protease A (*wprA*) gene encodes a 96 kDa polypeptide containing a signal peptide, propeptide and a protease which upon synthesis and export gives rise to two cell wall-bound proteins, CWBP23 and CWBP52 (Figure 1). The CWBP52 polypeptide has protease activity that is inhibited with PMSF, an inhibitor of serine proteases. The deletion of *wprA* does not result in any noticeable phenotype in terms of growth rate, cell morphology, sporulation or motility.

The *wprA* is characterised by the following characteristics; the polypeptide is cell-wall associated and is expressed during both exponential and stationary growth phases.

We decided to investigate the phenotype of a *wprA* deletion strain with particular reference to the secretion of heterologous protein.

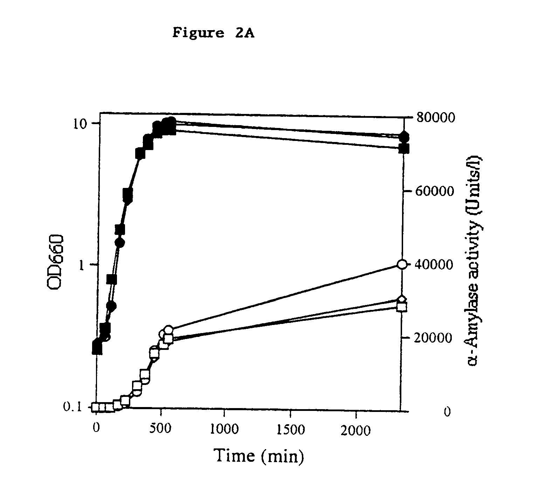

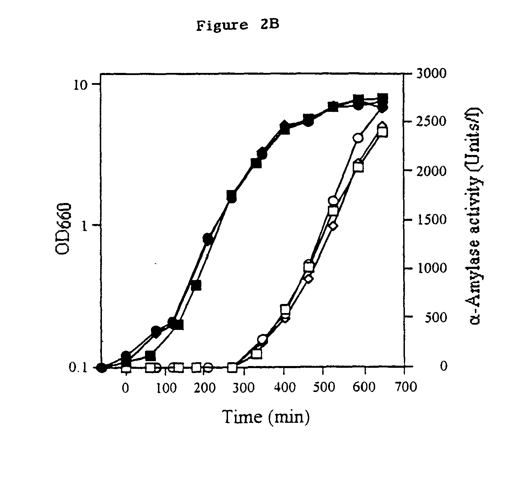

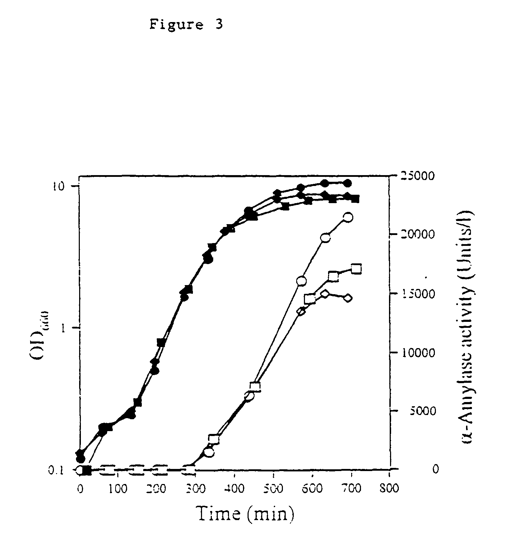

Using homologous recombination, we have generated a strain in which the *wprA* gene from the *B.subtilis* genome has been placed under the control of an inducible promoter. As mentioned above, this *wprA* controllable strain has no apparent phenotype, even in the absence of an inducer. Surprisingly, however, when the yield of a native bacillary model protein, the α-amylase from *Bacillus licheniformis* (AmyL) was compared in wild-type and *wprA* null strains there was an approximate 25% increase in the amount of α-amylase detected at the end of exponential growth, see figure 2A. The yield increased further to 41 % after prolonged incubation (Figure 3). Knocking out the *wprA* gene also resulted in an increase in the yield of an engineered α-amylase, AmyLQS50.5, when compared to the wild-type *wprA* strain (figure 2B). This suggests that switching off or deleting the *wprA* gene significantly enhances production of secreted homologous and heterologous proteins in *B. subtilis.*

The importance of the *wprA* gene with respect to the secretion of native, heterologous or recombinant protein is also shown by experiments that attempted to identify additional genes involved in the secretory pathway. We have used a *B. subtilis* strain, CJ278, transformed with a vector expressing a chimeric α amylase gene, please see materials and methods for details of the chimeric α amylase gene and *B.subtilis* strain construction. This strain showed decreased chimeric α-amylase secretion in comparison to the level of secretion by a wild type strain expressing wild type α amylase. We have used transposon mutagenesis using the mini-Tn 10 delivery vector pIC333. The integration of this transposon into the *B.subtilis* genome is random. The screen involved the identification of integration mutants that show elevated secretion of chimeric α-amylase. The mutant strains thus identified were analysed further by rescuing the plasmid DNA from the mutant strain and sequencing the flanking regions around the transposon integration site to determine the region of the *B.subtilis* genome in to which the transposon had integrated. The sequence of the plasmid rescued from the transposon mutant TK108 shows that the transposon had inserted into the *wprA* gene at position 2059.

It is therefore apparent that the absence of the WprA facilitates the secretion of native , heterologous or recombinant protein from *B.subtilis.*

It is therefore an object of this invention to develop a means of expressing recombinant protein in a prokaryotic expression system that allows the production of polypeptides in a biologically active form and at a high concentration.

It is a further object of the invention to develop a prokaryotic expression system that enables the secretion of recombinant protein into the culture medium to facilitate the purification of intact recombinant protein that retains biological activity.

Useful in a preferred embodiment is a bacterial strain wherein the *wprA* gene or its corresponding promoter has been altered by deletion and/or insertion and/or mutation and/or substitution so that either production of the said gene product is prevented or the gene product is non-functional to the extent that the use of the strain to produce native, heterologous or recombinant protein is facilitated.

In a preferred embodiment of the invention said bacterial strain, prior to said alteration, is wild-type for said *wprA* gene.

In yet a further preferred embodiment said bacterial strain is a Gram-positive bacterial strain.

In yet a still further preferred embodiment said bacterial strain is of the genus *Bacillus.*

Reference hereto the term bacterial strain includes reference to any bacterial strain but ideally a Gram-positive bacterial strain and, more ideally, but not obligatory, a bacterial strain of the genus *Bacillus.*

It will be apparent to those skilled in the art that where heterologous protein is to be produced the said bacterial strain will be transformed so as to include DNA encoding at least one selected native and/or heterologous and/or recombinant protein.

In a preferred embodiment of the invention the said strain is manipulated so that at least a part of the *wprA* gene is deleted. Ideally a significant amount of the gene is deleted, but in certain aspects of the invention the pre-sequence, or a part thereof, or alternatively, the pro-sequence, or a part thereof, or alternatively the serine protease sequence, or a part thereof is deleted. Alternatively, a selected alternative part of the gene may be deleted or a combination of selected parts may be deleted.

In an alternative embodiment of the invention genetic material may be inserted into the *wprA* gene at at least one selected location with a view to preventing expression of the gene or synthesis of at least a part of the functional protein product.

Alternatively, at least one selected point mutation may be provided in the said gene with a view to either preventing synthesis of the protein or preventing expression of the gene. For example, and without limitation, the reading frame of the gene may be altered so as to encode a stop codon thus preventing synthesis of a functional protein.

In a yet further preferred embodiment of the invention said *wprA* gene is altered by way of modification of an expression control sequence, ideally a promoter such that the promoter is made responsive to a specific signal, for example, the *wrpA* gene may be placed under the control of an inducible promoter such that expression of the *wrpA* gene product may be selectively controlled.

In a preferred embodiment of the invention the bacterial strain is of the genus *Bacillus* and is ideally the species *Bacillus subtilis* or its close relatives such as *B. amyloliquefaciens, B. licheniformis* and *B. stearothermophilus.*

It is noted that the *wprA* gene encodes a protein with specific domains attributed to its derived amino acid sequence. The polypeptide can be divided into a signal peptide, propeptide and protease polypeptide that have been accurately defined in Margot and Karamata (Microbiology 1996 142 3437-3444). The signal peptide is involved in targeting the *wprA* gene product to the secretory apparatus necessary for translocation across the cell membrane. The propeptide is likely to be a chaperone-type molecule involved in folding and maturation of the CWBP52 protein into a biologically active form. The propeptide is stable and may perform other important functions. The protease polypeptide will require the presence of both the pre and pro-sequences to be effectively targeted and to function efficiently.

Useful in a preferred embodiment is a bacterial strain, preferably of the genus *Bacillus*, and ideally of the species *B. subtilis* having a deletion in at least part of the sequence represented in Figure 1 from nucleic acid base pair +154 to +247 inclusive, or a corresponding part of a homologous gene.

In a preferred embodiment there is provided a *B. subtilis* strain deleted for a part of the *wprA* gene encoding the signal peptide of the coding sequence of the WprA precursor protein.

Useful in a preferred embodiment is a bacterial strain preferably of the genus *Bacillus* and ideally *B. subtilis,* having a deletion of, at least a part of, the sequence represented in Figure 1 from nucleic acid base pair +154 to +1392 inclusive, or a corresponding part of a homologous gene.

In a preferred embodiment the said bacterial strain may be modified, additionally or alternatively, by deletion of, at least part of, the DNA sequence presented in Figure 1 from +247 to +1392, or a corresponding part of a homologous gene.

In yet a further preferred embodiment said *B. subtilis* strain is deleted for that part of the *wprA* gene encoding, at least a part of, the polypeptide CWBP23.

Useful in a preferred embodiment is a bacterial strain preferably of the genus *Bacillus* and ideally the species *B. subtilis* having a deletion of, at least a part of, the sequence represented in figure 1 from nucleic acid base pair + 1392 to +2835 inclusive, or a corresponding part of a homologous gene.

In a preferred embodiment there is provided a *B*. *subtilis* strain deleted for that part for the *wprA* gene encoding, at least a part of, the CWBP52 polypeptide.

In yet a further preferred embodiment of the invention said bacterial strain is deleted for at least part of the sequence represented in Figure 1 from nucleic acid base +247 to + 2835.

In yet a further preferred embodiment of the invention there is provided a *B.subtilis* strain deleted for part of the *wprA* gene encoding either or both the proposed propeptide (CWBP23) or the scrine protease (CWBP52).

In yet a further preferred embodiment the said strain includes a deletion in that part of the gene encoding a polypeptide that is a serine protease.

In one aspect of the invention there is provided a method for producing a desired polypeptide, wherein a microbial strain as indicated above is used for the production of said polypeptide by growing said strain under conditions conducive to the production of said polypeptide of interest, and recovering said polypeptide of interest.

It is envisaged that the polypeptide of interest may be endogenous or heterologous to the strain in question.

According to an embodiment of this aspect said microbial strain is used as a host into which a polynucleotide construct encoding said polypeptide of interest is brought in a functional manner by which said strain is capable of expressing said polypeptide.

The polynucleotide construct may be transferred into the strain by any method known in the art, such as transformation, conjugation, or protoplast transformation The construct may be a plasmid or any other vector suitable for the specific method used for introducing said polynucleotide construct into the microbial cell.

In the cell said construct may be present on a plasmid or integrated into the chromosome of said strain. Furthermore, it may be present as a single copy or in multiple copies provided by either amplification or by multiple integrations.

The polypeptide may be a peptide or protein of any type, especially an industrial enzyme. Said enzyme may be any enzyme that can be produced in a strain according to the invention, such as a carbonyl hydrolase, carbohydrase, protease, lipase, amylase, cellulase, oxido reductase, glucoamylase, or esterase.

In essence the invention provides a bacterial strain, ideally a *B. subtilis* strain, altered by mutation, substitution, insertion or deletion either entirely or in part, for the *wprA* gene, or a homologue thereof, which gene encodes a cell wall-associated serine protease. It is surprising that given the presence of additional extracellular protease genes in *B. subtilis* that deletion of the single copy *wprA* gene should result in a significant effect on the production of both endogenous and heterologous recombinant protein.

An embodiment of the invention will now be described by way of example only with reference to the following figures wherein:
Figure 1 shows the nucleotide sequence of the region of the *B. subtilis* genome containing the *wprA* gene and amino acid sequence of its product WprA;
Figure 2A represents yields of α-amylase released into culture medium. Closed symbols represent growth and open symbols α-amylase activity. *B.subtilis* strains KS408 (**■**), KS408 *wprA* :: pMutin2 with (**◆**) or without (•) IPTG (10 mM);
Figure 2B is similar to the experiment described in Figure 2A but the *B. subtilis* strains are expressing a recombinantly manufactured chimeric α-amylase (AmyLQS50.5). Experimental details relating to induction of the *wprA* gene product are as in Figure 2A and are described in detail in the materials and methods;

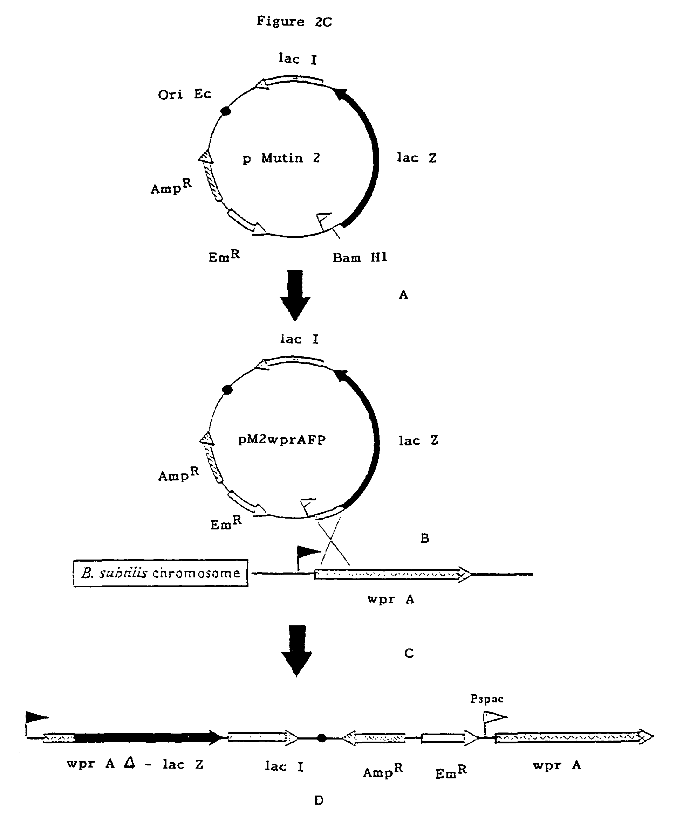

Figure 2C is a diagrammatic representation of the construction of a B.subtilis strain encoding an inducible *wprA* gene. Closed flags represent the native *wprA* promoter (P*wprA*), and open flags the IPTG- inducible promoter (Pspac). Ori Ec; *E.coli* origin of replication; A represents the sub-cloning of a *wprA* PCR 5' fragment into the BamH1 site in pMutin2; B represents a single cross-over event between pM2*wprA*FP and the *B.subtilis wprA* gene; C represents the integration of pM2*wprA*FP into the *B.subtilis* chromosome by homologous recombination; and D represents the structure of the *B.subtilis* chromosome after the integration event.
Figure 3 represents a comparison of AmyL production in a wild type *B.subtilis* strain and a strain with a *wprA* gene product under the control of an IPTG inducible promoter in the absence or presence of IPTG. Cultures of *B.subtilis* were grown to stationary phase and AmyL activity was compared during exponential growth phase and after approximately 30 hours in stationary phase;

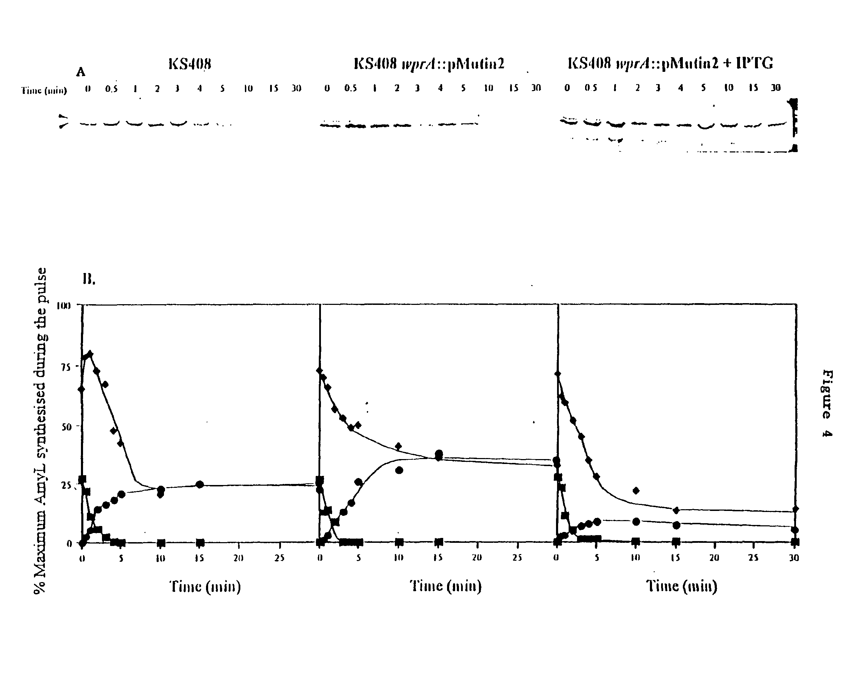

Figure 4 represents the secretion kinetics of AmyL from exponetially growing *B.subtilis* in the presence and absence of the *wprA* gene products. Representative data from pulse-chase experiments carried out on strains KS408 and KS408*wprA* ::pMutin2+/- 10mM IPTG. (A) Autoradiographs of pulse-chased AmyL following immunoprecipitation and SDS-PAGE. The top panel .......shows precursor (p) and mature(m) immunoprecipitated from whole culture samples and the bottom panel mature AmyL (m) released into culture medium. Quantification by phospho-imaging of the different forms of AmyL at time intervals following the chase; AmyL precursor ( ) and mature AmyL in whole cultures samples (**◆**), and mature AmyL released into growth medium (•). The amount of each form of AmyL is expressed as a percentage of the total AmyL (precursor + mature) synthesized during the pulse;....

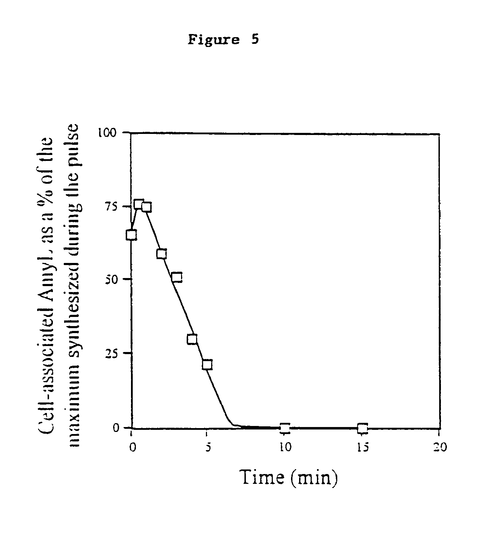

Figure 5 represents cell-associated degradation of AmyL as determined by subtracting the data for the released mature AmyL from that obtained in the whole culture samples. The amount of AmyL at each interval is expressed as a percentage of the maximum amount of AmyL (precursor + mature) synthesized during the pulse;

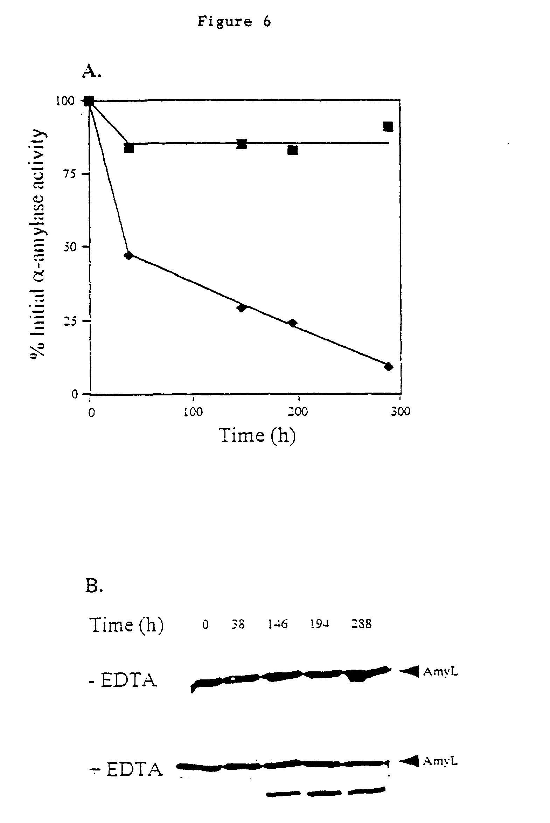

Figure 6 represents the stability of AmyL in spent culture medium at 4°C. (A) α-Amylase activity at time intervals in the absence (■) or presence (◆) of 10mM EDTA. (B) western blots of AmyL in spent culture medium at time intervals in the absence and presence of 10mM EDTA; and

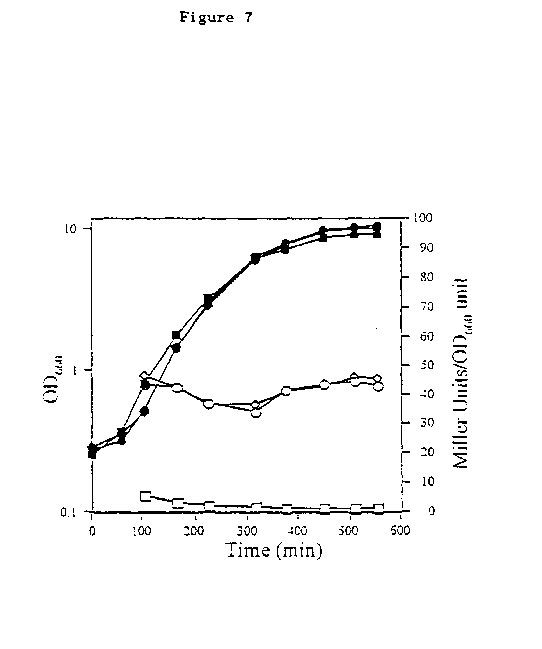

Figure 7 represents the transcriptional activity of the *wprA* gene using the *wprA*Δ-lacZ transcriptional fusion. Growth (closed symbols) and β-galactosidase activity(open symbols) were measured in cultures of *B.subtilis* KS408 (■) and KS408*wprA* ::pMutin2 with (◆) or without (**●**) IPTG (10mM)

Table 2 shows the production of the AmyL α-amylase by *B. subtilis* in the absence or presence of the *wprA* gene product in a very nutritious, industrial type medium in an extended batch fermentation. Each strain was grown for approximately 7 days at 37°C and α amylase activity was measured in the supernatant at the end of this period. Experimental details are given in the materials and methods.

### MATERIALS AND METHODS

The initial analysis of the involvement of the *wprA* gene product in secretion of endogenous and heterologous recombinant protein dealt with the construction of a *B. subtilis* strain in which the single copy *wprA* gene promoter was substituted by an IPTG inducible promoter. In the absence of IPTG the expression of the *wprA* gene is repressed. Upon addition of IPTG the *wprA* gene is induced.

Alternatively the *wprA* gene can be entirely or partially deleted from the *B.subtilis* genome as detailed in preceding description and the following methods.

### Bacterial strains

The bacterial strains used are shown in table 1.

**Table 1. Bacterial strains**

| Strain | Comments |
|---|---|
| *E. coli* XL1-Blue | - |
| *B.subtilis* DN1885 xy1R::pKS405B | Encodes chimeric α-amylase AmyLQS50.5 |
| B. subtilis DN1885 xy1R::pKS408 | Encodes wild type α-amylase, AmyL |
| *B. subtilis* DN1885 xy1R::pKS405B, wprA:: pM2 wprAFP. | B.subtilis DN1885 xy1R:: pKS405B with IPTG-inducible wprA |
| *B. subtilis* DN1885 xy1R:: pKS408 | B. subtilis DN1885 xy1R::pKS408 |
| wprA::pM2 wprAFP | with IPTG-inducible wprA |
| *B.subtilis* DN1885 Novo Nordisk J.of Bacteriology 172:4315-4321, 1991 | α-amylase (*amyE*) negative derivative of *B.subtilis* RUB200 |

### Growth media

*B.subtilis* and *E.coli* were maintained on antibiotic medium number 3 (Difco) solidified with 1.5% w/v agar and containing 1% w/v soluble starch. Batch cultures were grown in 2xYT broth which contained; tryptone (1.6%w/v), yeast extract (1.0%w/v) and NaCl (0.5%w/v). Where required antibiotics were included in the growth media at the following final concentrations: chloramphenicol g/ml, ampicillin g/ml and erythromycin g/ml. Xylose (1% w/v) was added to induce the synthesis of α-amylase from a xylose-inducible promoter. The comparison of α-amylase production in B.subtilis wild type and a strain deleted for the *wprA* gene product was also done in an industrial type medium containing potato starch (100g/1), barley flour (50g/1), BAN 5000 SKB (0.1g/l), sodium caseinate (10g/l), soy bean meal (20g/l), Na₂HPO₄.12 H²0 (9g/l) and pluronic (0.1g/l). For the wild-type strain the medium was supplemented with 6 g/ml chloramphenicol and 0.2% xylose. For the *wprA* deletion strain medium was supplemented with 6 g/ml chloramphenicol 5 g/ml erythromycin and 0.2% xylose.

### DNA manipulations and bacterial transformation

Restriction digestion, DNA fragment purification, ligation and transformation of *E.coli* were carried out as described previously (Sambrook et al., 1989). Chromosomal DNA was isolated from *B.subtilis* using the IGi Genomic extraction kit (Immunogen International). PCR was carried out with Taq DNA polymerase (Appligene) using *B.subtilis* DN1885 chromosomal DNA as the template. Plasmid DNA was purified from *E.coli* and *B.subtilis* with the Tip-100 plasmid extraction kit (Qiagen). Oligonucleotide primers for PCR were synthesized using a Beckman Oligo 1000. *B.subtilis* was grown to competence and transformed with integrative plasmids.

### α-Amylase assay

The quantity of secreted α-amylase was quantified using the Phadebas α-amylase assay kit (Kabi Pharmacia). The cells from culture samples were pelleted by microcentrifugation and the α-amylase activity in the supernatant determined as described by the manufacturer.

### Construction of a Strain Encoding An Inducible wprA

To determine whether the products of the *wprA* gene are involved in the co-or post-translocational degradation of AmyL, we constructed a strain of *B.subtilis* in which an intact copy of the gene is under the control of the isopropylthio-β-D- galactoside (IPTG) -inducible Pspac promoter. The constructs were made using the pMutin2 integration vector. A 357-base pair DNA fragment corresponding to the 5' end of the *wprA* gene was amplified by PCR from *B.subtilis* KS 408 chromosomal DNA using oligonucleotide primers WPR-F (5' GCGCGCGCGGATCCGGGATAACATGAAACGC 3') and WPR-R (5' GCGCGCGCGGATCCCCATCCTCCGCTGTG 3'). This fragment was cloned into the unique BamH1 restriction site of pMutin2 using *E.coli* XL1-Blue as the host.

The resultant plasmid, pM2*wprA*FP, was used to transform *B.subtilis* KS408 to produce strain KS408 *wprA* ::pMutin2. Since the *wprA* gene of KS408 *wprA* ::pMutin2 is under the control of the Pspac promoter, its expression can be controlled by the presence or absence of IPTG. Additionally, a transcriptional fusion (*wprA* Δ-lacZ) was created between the native *wprA* promoter and lacZ to allow the expression of *wprA* to be monitored via β-galactosidase activity, Figure 7.

Construction of a WprA Negative Strain By Deletion in the *B.subtilis* DN1885 *wprA* Gene

Plasmid pCJ791, encoding a N-terminal fragment (bp 133 to bp 615) and a C-terminal fragment (bp 2364 to bp 2781) of the wprA gene from *B.subtilis* DN1885, was constructed in four steps:
i) a 382 bp N-terminal fragment of the wprA gene was amplified by PCR from *B.subtilis* DN1885 using oligonucleotide primers
   CLJe7
   (5'-GGAATTCCAAAGCTGCAGCGGCCGGCGCG-3'),
   and CLJe8
   (5'-GAAGATCTCGTATACTTGGCTTCTGCAGCT-3').
   This fragment had a EcoRI restriction site at the 5'-end and a BglII restriction site at the 3'-end. Simultaneously, a 419 bp C-terminal fragment of the *wprA* gene was amplified by PCR from *B. subtilis* DN1885 using oligonucleotide primers
   CLJe9
   (5'-AGATCTGGTCAACAAGCTGGAAAGCACTC-3')
   and CLJe10
   (5'-CCCAAGCTTCGTGACGTACAGCACCGTTCCGGC-3').
   This C-terminal fragment had a BglII restriction site at the 5'-end and a HindIII restriction site at the 3'-end.
ii) The two DNA fragments, encoding the N- and C-terminal sequences of the *wprA* gene, were digested with BgIII restriction enzyme and ligated to form a fragment of 801 bp. Using oligonucleotide primers CLJe7 and CLJe10, the 801 bp fragment was amplified by PCR from the ligation mixture. The amplified 801 bp fragment was digested with EcoRI and HindIII restriction enzymes.
iii) A 4.4 kbp EcoRI to HindIII fragment from plasmid pSJ2739 (described in patent application WO 96/23073, figure 6) was purified and used as vector for the 801 bp fragment. This plasmid is based on the pE194 origin of replication which means that the replication of the plasmid is temperature sensitive.
iv) The two EcoRI to HindIII fragments (801 bp and 4.4 kbp) were ligated and plasmid pCJ791 was obtained by selecting for resistance to erythromycin at 28°C using *B. subtilis* DN1885 as host strain.

Plasmid pCJ791 was integrated into the chromosome of *B.subtilis* DN1885 by selecting for resistance to erythromycin at 37°C. Since pCJ791 is based on the pE194 origin of replication, transformants were selected in which the plasmid had integrated into the chromosome by homologous single crossover recombination between one of the plasmid *wprA* sequence and the corresponding chromosomal *wprA* sequence. Two types of integrant strains could be the result of the integration event, i) the integrated plasmid followed by the wild-type *wprA* gene or ii) the wild-type *wprA* gene followed by the integrated plasmid. For the construction of a clean *wprA* deletion strain, both types of integrant strain could be used. Hence, the integration event was not investigated further.

A clean *wprA* deletion strain was then constructed by homologous single crossover resulting in release of the integrated plasmid. There were two ways in which the plasmid could be released from the chromosome i) by the same recombination as the plasmid was integrated or ii) by recombination between the sequence that was not involved in the integration event. For the first case, the resulting strain would have a wild-type *wprA* gene on the chromosome. If the second case occurs, the resulting strain would have a deleted *wprA* gene on the chromosome and, by that, the wanted event. In order to release the integrated plasmid, twelve transformants were inoculated in TY-medium without selection and cultivated at 28°C over night. The cultures were once again inoculated in fresh TY-medium and cultivated at 28°C over night. After three rounds of inoculation, the cultures were spread on LB-plates without selection and, subsequently, the obtained colonies were screened for sensitivity to erythromycin. Twenty-four colonies sensitive to erythromycin (Erm^{s}) were checked for the presence of a deleted *wprA* gene by PCR directly on colony using oligonucleotide primers CLJe7 and CLJe10. Four of these Erm^{s} colonies had a deleted *wprA* gene on the chromosome. The authenticity of the *B. subtilis* DN1885Δ*wprA* strain was confirmed by Southern Blot Hybridisation.

### Isolation of a wprA mutant with increased level of secreted chimeric α-amylase

In a previous study where the aim was to investigate how the net charge of proteins affected their passage through the negatively charged cell wall in *Bacillus subtilis* it was observed that both wild type AmyL (α-amylase from *Bacillus licheniformis*) and chimeric variants are subject to co-and/or post-translocational degradation. Protease(s) responsible for this degradation are likely to be associated with the cytoplasmic membrane or cell wall, since the proteolytic degradation occurs on the outer surface of the cytoplasmic membrane ("Construction and use of chimeric α-amylase to study protein secretion in *B.subtilis"* PhD thesis by Keith Stephenson, University of Newcastle Upon Tyne, 1996; " Secretion of chimeric α-amylase from *Bacillus subtilis"* PhD thesis by Christina Lund Jensen, Technical University of Denmark, 1997). In a search of factors involved in this degradation, a screening system based on the *B.subtilis* strain CJ278, expressing the chimeric α-amylase (AmyLQS55-6) was set up. A mutant library was prepared by transposon mutagenesis, and subsequently screened for mutants with increased halo formation on amylase screening plates.

### Construction of a chimeric α-amylase, AmyLQS55-6:

In the following an overview of the steps involved in the construction of the chimeric α-amylase AmyLQS55-6 is given. A detailed description is given in: "Secretion of chimeric α-amylases from *Bacillus subtilis."* Ph.D thesis by Christina Lund Jensen, Technical University of Denmark, 1997.

The chimeric α-amylase, AmyLQS55-6 was constructed by swapping specific blocks of the mature portion of the α-amylase from *B. licheniformis* (AmyL) with the corresponding blocks from the α-amylase from *B.amyloliquefaciens* (AmyQ) or from *B.stearothermophilus* (AmyS). The individual DNA blocks were constructed by using a PCR-based in vitro gene splicing method, the SOE method (splicing by overlap extension, Horton et al. Gene 77, 61-68, 1989). The amyL gene has a unique Pst1 site located within the signal sequence and a unique HindIII site 3' to the transcription terminator. The amyLQS55-6 gene was therefore designed as in-frame PstI to HindIII DNA fragments encoding the mature part of the α-amylase. The amyLQS55-6 gene was divided into 3 separate DNA blocks, with block 1 covering a PstI-BamHI fragment, block 3 a KpnI-SaII fragment and block 4 a SalI-HindIII.

The properties of each block is given below, with the base numbers calculated in relation to the start codon in each gene.
Block 1: bp 79-132 amyL, bp 151-174 amyS, bp 157-198 amyQ, bp 199-213 amyL,
Block 3: bp 562-993 amyL, bp 1018-1095 amyS, bp 1072-1095 amyL
Block 4: bp 1096-1221 amyL, bp 1237-1419 amyS, bp 1411-1542 amyQ, bp 1537-1798 amyL

Between block 1 and 3 is a wild type amyL block covering bp 214-561.

The individual blocks were cloned into pUC19 in the correct order to produce the PstI to HindIII gene fragment encoding the mature AmyLQS55-6 protein.

For assembly of the individual blocks advantage was taken of the unique restrictions sites generated at their ends (created by the SOE method).

### Expression of the chimeric α-amylase, amyLQS55-6

The amyLQS55-6 gene was integrated into the *B.subtilis* chromosome by homologous recombination between a plasmid encoded and a chromosomal encoded copy of the xylR-gene. The assembled amyLQS55-6 gene was cloned into plasmid pCJ92. Plasmid pCJ92 is derived from pSX63 which encode for a xylose-inducible promoter system (for detailed information about the construction of plasmid pCJ92; Secretion of chimeric α-amylases from *Bacillus subtilis.* Ph.D thesis by Christina Lund Jensen, Technical University of Denmark, 1997). The EcoRI to BgIII fragment of the amyLQS55-6 gene encoded by pCJ92 was cloned into pUC19 EcoRI and BamH1 restriction sites in *E.coli* SJ2, resulting in plasmid pCJ272. Plasmid pCJ272 does not contain an origin of replication that is functional in *B.subtilis.* The integration plasmid, pCJ272, was introduced into the *B.subtilis* strain DN1885 and transformants were obtained by selection for chloramphenicol resistant colonies. The plasmid was integrated into the chromosome of DN1885 by a single, homologous recombination between the plasmid encoded and chromosomally encoded copies of the xylR-gene.

The integration of the α-amylase expression cassette into the chromosome of DN1885 resulted in a stable system which allowed the production of α-amylase to be induced in the presence of xylose.

### Screening system:

The screening system for identification of mutants with an improved secretion of α-amylase is based on the *B.subtilis* strain CJ278 (DN1885 xy1R::pCJ272) harbouring the gene encoding the chimeric α-amylase AmyLQS55-6. In comparison to the wild type AmyL, the level of α-amylase secretion from strain CJ278 is about 1%, meaning that CJ278 gives rise to colonies with a small and well-defined halo of starch degradation on plates. Therefore, it was considered an ideal candidate for screening of yield mutants.

### Mutagenesis protocol:

For transposon mutagenesis of strain CJ278, the mini-Tn10 delivery vector pIC333 was used (Steinmetz, M. and Richter, R. 1994. J. Bacteriol. 172:5019). Outside the transposon, this plasmid carries a modified transposase gene conferring relaxed target specificity, a thermosensitive origin of replication and an erythromycin resistance gene for selection at permissive temperatures. The 2.2 kbp transposon encodes the spectinomycin resistance gene and the pUC8 origin of replication, allowing replication in *E.coli.* Plasmid pIC333 was transformed into strain CJ278 and erythromycin resistant transformants were inoculated to TY-medium supplemented with 0.4% glucose and spectinomycin (120 µg/ml) and grown over night at 28°C. The over night culture was diluted 1/100 in TY-medium supplemented with 0.4% glucose and spectinomycin (120 µg/ml). After 3 hours of cultivation the temperature was shifted to 37°C (which is the restrictive temperature) and the culture was cultivated for an additional 4 hours. Aliquots of the culture were plated on LB-amylopectin (coupled to Cibacrone red) plates supplemented with 0.4% glucose, 0.01 M phosphate pH 7, 0.2% xylose and 120 *µ*g/ml spectinomycin and incubated over night at 37°C. Colonies with a distinctly larger halo, indicating a higher amount of secreted α-amylase, appeared with a frequency of 1/150. One such transposon mutant, forming a larger halo of starch degradation than the parent strain, was strain TK108.

The mini-Tn10 transposon and its flanking regions from strain TK108 were rescued, taking advantage of the pUC origin of replication present in the transposon. The TK108 chromosome was totally digested with EcoRI, and religated with T4 DNA ligase. The ligation mixture was transformed into *E.coli* SJ2 (Diderichsen et al, 1990. J. Bacteriology 172, 4315-4321), selecting for spectinomycin resistance. Plasmid DNA from spectinomycin-resistant transformants was used for DNA sequencing. The DNA sequences was determined by the dideoxy chain termination method (Sanger et al 1997) and by using mini-Tn10 specific primers: 5'- CCA ATA CGC AAA CGC CCT CTC-3' and 5'- TAG TGA CAT TTG CAT GCT TC- 3', which correspond to position 137-117 and 2181-2200, respectively, on the mini-Tn10 transposon sequence.

The sequence of the plasmid rescued from transposon mutant TK108 shows that the transposon had inserted into the *wprA* gene, at position 2059.

### Results and Discussion

The development of efficient alternative methods of native, heterologous or recombinant protein manufacture is obviously desirable. It is apparent that not all heterologous protein can be produced in a soluble, biologically active form therefore methods that facilitate the production of such proteins are continuously being designed.

We have taken the approach to develop the genus *Bacillus* and its close relatives as an alternative host cell for the production of the both native and heterologous and recombinant protein. These bacteria have considerable advantages over other species due to their ease of growth in batch cultures and their rapid rate of cell division and furthermore their ability to secrete proteins into the culture medium at high concentrations. Furthermore, we have decided to focus on the development of an expression system that produces secreted soluble protein into the culture medium to facilitate the purification of such proteins from contaminating endogenous bacterial proteins and other macromolecules. A major problem with this methodology is that many bacterial systems actively secrete proteases into the culture medium that degrade proteins in the immediate environment of the bacterial cells. Some *B.subtilis* strains have been engineered to delete these genes from the bacterial genome to reduce the loss of protein through proteolytic activity. However, this can still lead to reduced yields of intact protein due to, amongst other things , the release of intracellular proteases into the growth media as strains that are multiply deficient in extracellular proteases become prone to lysis thereby releasing cellular contents into the surrounding growth media.

Additionally, we have undertaken pulse-chase labelling experiments to identify the sites at which proteolysis of secreted proteins occurs. The amount of mature Amy L (a model secreted protein) released into the culture medium can be seen to increase with time until it reaches a constant level. This level represents only approximately 25% of the total Amy L synthesized, an amount consistent with the AmyL remaining in whole culture samples. This means that 75% of the initially synthesized AmyL is degraded. By determining the proportion of AmyL that remains cell-associated at each time point after the addition of the chase solution, by subtracting the amount of AmyL released from that observed in whole culture samples, it was possible to determine that AmyL degradation occurred in a cell-associated location and within 7 minutes of the addition of chase, Figure 6. This data suggests that the observed degradation of AmyL occurs during or shortly after translocation across the membrane and in a cell-associated location.

The *wprA* gene of *B. subtilis* encodes a cell wall-associated serine protease. The *wprA* gene product is composed of a presequence (signal peptide) to assist in targeting the protease to the secretory apparatus, a prosequence which produces a stable 23kDa protein product most likely with chaperone type activity and a 52kDa serine protease. We have engineered the *wprA* gene by placing it under the control of an IPTG inducible promoter element. This allows the expression of *wprA* to be strictly regulated, simply by the presence or absence of IPTG in the growth medium. When a *B.subtilis* strain which showed decreased α-amylase production is randomly mutagenised with the mini Tn 10 transposon, integrant mutants with enhanced α-amylase production were identified. Sequence analysis of rescued Tn 10 DNA revealed the integration site to be the *wprA* gene. We have also created a *B.subtilis* strain that has been genetically engineered to completely delete the *wprA* gene from the genome.

We have taken wild type *B. subtilis* strain DN1885 xy1R::pKS405B transformed with a chimeric α-amylase gene and strain DN1885 xy1R::pKS408 which is transformed with wild-type α-amylase and replaced the *wprA* promoter sequence with an IPTG inducible promoter contained in the plasmid pM2*wprA*FP (see materials and methods).

The activity of secreted wild-type α-amylase was assessed in *B. subtilis* cultures in the presence or absence of 10mM IPTG. A culture in which *wprA* was expressed from its native promoter was used as a controlled culture. Figure 2A indicates that the growth rate and kinetics of a *B. subtilis* strain is not significantly affected by the absence of WprA protein (no IPTG added), as measured by culture optical density. However, in the absence of 10mM IPTG there is approximately a 25% increase in α-amylase activity in the culture medium compared to the wild-type strain in which the *wprA* gene is expressed from its native promoter (Figure 2A). Figure 2B indicates a comparable effect on the production of a chimeric -amylase.

The yield of native α-amylase in the culture medium was also assessed in stationary phase cultures of *B. subtilis* wild-type or IPTG-inducible *wprA* genes, (figure 3). The strains were grown for approximately 39 hours at which time cultures had been in stationary phase for approximately 30 hours. In the absence of IPTG the yield of α-amylase in the culture medium had increased by approximately 40% when compared to a strain expressing *wprA* from its native promoter. In contrast, the yield of α-amylase from KS408 *wprA* ::pMutin2 in the presence of IPTG *(wprA* on) was lower and on transition to stationary phase the yield of α-amylase was 95% that of KS408.

Additionally, when strains are grown in a rich industrial type medium in an extended batch fermentation culture, there is approximately a 78% increase in α-amylase activity in the absence of the WprA protein.

These data demonstrate that expression of *wprA* markedly influences the yield of released α-amylase.

We have additionally used coupled pulse-chase and immunoprecipitation techniques to investigate the secretion kinetics of AmyL in KS408 and KS408 *wprA* : :pMutin2. Cultures were grown to exponential phase (OD₆₀₀~ 0.6) and pulse-chased with L-(³⁵S) methionine. Following inmmunoprecipitation and subsequent SDS-PAGE, both precursor and mature forms of AmyL were visualised by autoradiography, Figure 4. In the case of KS408, the processing of the AmyL precursor to the mature form was rapid; in samples taken immediately following the chase (0 min) only 27% of the total AmyL ( precursor + mature) synthesized during the pulse was in precursor form, Figure 4. Processing was complete by 5 min post-chase when all the α-amylase was in mature form. The amount of mature AmyL in the whole culture sample (cells + growth medium peaked at 1 minute, after which time it declined until it reached a constant level of approximately 25% of the maximum detected, representing a significant loss of newly synthesized α-amylase during or shortly after translocation across the cytoplasmic membrane.

The involvement of the WprA protein in the secretion of protein from *B.subtilis* is also confirmed by the mutant screen undertaken to identify mutated strains that show enhanced secretion of a chimeric α-amylase. The mini-Tn 10 transposon randomly integrates into bacterial genomic DNA thereby creating insertional mutations if the transposon does not integrate in an essential gene. The mutant strain TK108 shows increased secretion of chimeric α-amylase when compared to a wild type control strain. The mini-Tn 10 delivery vector was recovered from TK108 genomic DNA and the flanking regions surrounding the vector was sequenced to determine the site of integration. The transposon had integrated at position 2059 of the *wprA* gene. The disrupted *B.subtilis* strain TK108, showed elevated α amylase secretion as monitored by the size of the halo produced around TK108 when compared to CJ278(wild type) on starch agar plates.

In conclusion we have shown that switching off or disabling or deleting the single copy *wprA* gene significantly increases the yield in the culture medium of native, heterologous or recombinant proteins from *B. subtilis.* Importantly *B. subtilis* extracellular proteases are still actively secreted into the growth medium indicating that a major contributing factor in the production of increased yields of secreted α-amylase is removal of the cell wall associated protease encoded by the *wprA* gene.

**Table 2**

| Relative yields of α-amylase is wild-type and *wprA* IPTG-inducible *B. subtilis* strains. | |
|---|---|
| *B. subtilis* DN1885 xylR::pKS408 | *B. subtilis* DN1885 xylR::pKS408 *wprA*::pM2 wprAFP |
| 100 | 178 |

## Claims

1. A method for producing a native and/or heterologous and/or recombinant polypeptide from a microbial strain having a deletion and/or insertion and/or mutation and/or substitution in a *wprA* gene or its corresponding promoter such that the production and/or function of the gene product encoded by the *wprA* gene is affected in a manner that facilitates production of said polypeptide comprising;
i) growing said strain under conditions conducive to the production and secretion of said polypeptide(s); and
ii) recovering said polypeptide(s) from growth media and/or said bacterial strain.

2. The method according to claim 1, wherein said microbial strain is a Gram positive strain.

3. The method according to claims 1 or 2, wherein said microbial strain is of the genus *Bacillus.*

4. The method according to any of claims 1-3, wherein said bacterial strain is *B.subtilis, B.amyloliquifaciens, B.licheniformis,* or *B.stearothermophilus.*

5. The method according to any of claims 1-4, wherein said polypeptide is a carbonyl hydrolase, carbohydrase, protease, lipase, amylase, cellulase, oxidoreductase, glucoamylase, or esterase.

6. The method according to any of claims 1-5, wherein a *wprA* gene has been altered by deletion and/or insertion and/or mutation and/or substitution to prevent expression of said gene or to prevent synthesis of at least part of the functional protein product.

7. The method according to claim 6, wherein said wprA gene is mutated by at least one selected point mutation.

8. The method according to claim 6 or 7, wherein said *wprA* gene has been altered in an expression control sequence.

9. The method according to any of claims 6-8, wherein said *wprA* gene has been altered by insertion of genetic material into said *wprA* gene.

10. The method according to claim 8, wherein said expression control sequence is a promoter.

11. The method according to claim 10, wherein said alteration results in the provision of an inducible promoter to selectively control expression of the *wprA* gene.

12. The method according to any of claims 6-8, wherein said bacterial strain is *B.subtilis,* containing a deletion of at least part of the sequence represented in Figure 1 from nucleic acid base +154 to +247.

13. The method according to claim 12, wherein said *Bacillus* strain is deleted for part of the *wprA* gene encoding the signal sequence of the WprA precursor protein.

14. The method according to any of claims 6-8, wherein said bacterial strain is *B.subtilis,* containing a deletion of at least part of the sequence represented in Figure 1 from nucleic acid base +154 to +1392.

15. The method according to any of claims 6-8 or 13, wherein said deletion comprises at least part of the sequence represented in Figure 1 from nucleic acid base +247 to +1392.

16. The method according to claim 14, wherein the *wprA* gene is deleted for at least part of the *wprA* gene encoding CWBP23.

17. The method according to any of claims 6-8, wherein said bacterial strain is *B.subtilis,* containing a deletion of at least part of the sequence represented in Figure 1 from nucleic acid base +1392 to +2835.

18. The method according to claim 17, wherein the *wprA* gene is deleted for at least part of the *wprA* gene encoding CWBP52.

19. The method according to claims 6-8, wherein said bacterial strain is *B.subtilis,* containing a deletion of at least part of the sequence represented in Figure 1 from nucleic acid base +247 to + 2835.

## Patentansprüche

1. Verfahren zum Herstellen eines nativen und/oder heterologen und/oder rekombinanten Polypeptides aus einem Mikrobenstamm, der eine Deletion und/oder Insertion und/oder Mutation und/oder Substitution im *wprA*-Gen oder seinem entsprechenden Promotor aufweist, so dass die Produktion und/oder Funktion des durch das *wprA*-Gen kodierten Genprodukts ein einer Art beeinflusst ist, die die Herstellung des Polypeptides erleichtert, umfassend
(i) Wachsen des Stamms unter Bedingungen, die der Produktion und Sekretion des/der Polypeptide/s förderlich ist/sind, und
(ii) Wiedergewinnen des/der Polypeptide/s aus dem Wachstumsmedium und/oder dem Bakterienstamm.

2. Verfahren nach Anspruch 1, wobei der Mikrobenstamm ein Gram-positiver Stamm ist.

3. Verfahren nach Anspruch 1 oder 2, wobei der Mikrobenstamm vom Genus *Bacillus* ist

4. Verfahren nach einem der Ansprüche 1 bis 3, wobei der Bakterienstamm *B.subtilis, B.amyloliquifaciens, B.licheniformis* oder *B. stearothermophilus* ist.

5. Verfahren nach einem der Ansprüche 1 bis 4, wobei das Polypeptid eine Carbonylhydrolase, eine Carbohydrase, eine Protease, eine Lipase, eine Amylase, eine Cellulase, eine Oxidoreduktase, eine Glucoamylase oder eine Esterase ist.

6. Verfahren nach einem der Ansprüche 1 bis 5, wobei das *wprA*-Gen durch Deletion und/oder Insertion und/oder Mutation und/oder Substitution geändert wurde, um die Expression des Gens zu verhindern oder die Synthese mindestens eines Teils des funktionellen Proteinprodukts zu verhindern.

7. Verfahren nach Anspruch 6, wobei das *wprA*-Gen durch mindestens eine ausgewählte Punktmutation mutiert wird.

8. Verfahren nach Anspruch 6 oder 7, wobei das *wprA-*Gen in einer Expressionskontrollsequenz verändert wurde.

9. Verfahren nach einem der Ansprüche 6 bis 8, wobei das *wprA*-Gen durch eine Insertion eines genetischen Materials in das *wprA*-Gen verändert wurde.

10. Verfahren nach Anspruch 8, wobei die Expressionskontrollsequenz ein Promotor ist.

11. Verfahren nach Anspruch 10, wobei die Änderung in der Bereitstellung eines induzierbaren Promotors resultiert, um die Expression des *wprA*-Gens selektiv zu kontrollieren.

12. Verfahren nach einem der Ansprüche 6 bis 8, wobei der Bakterienstamm *B.subtilis* ist, der eine Deletion mindestens eines Teils der Sequenz enthält, die in Figur 1 von den Nucleinsäurebasen +154 bis +247 dargestellt ist.

13. Verfahren nach Anspruch 12, wobei der Bacillus-Stamm hinsichtlich eines Teils des *wprA*-Gens deletiert ist, der die Signalsequenz des *wprA*-Precursorproteins kodiert.

14. Verfahren nach einem der Ansprüche 6 bis 8, wobei der Bakterienstamm *B.subtilis* ist, der eine Deletion mindestens eines Teils der Sequenz enthält, die in Figur 1 von den Nucleinsäurebasen +154 bis +1392 dargestellt ist.

15. Verfahren nach einem der Ansprüche 6 bis 8 oder 13, wobei die Deletion mindestens einen Teil der Sequenz umfasst, die in Figur 1 von den Nucleinsäurebasen +247 bis 1392 dargestellt ist.

16. Verfahren nach Anspruch 14, wobei das *wprA*-Gen hinsichtlich mindestens eines Teils des *wprA*-Gens deletiert ist, der CWBP23 kodiert.

17. Verfahren nach einem der Ansprüche 6 bis 8, wobei der Bakterienstamm *B.subtilis* ist, der eine Deletion in mindestens einem Teil der Sequenz enthält, die in Figur 1 von den Nucleinsäurebasen +1392 bis +2835 dargestellt ist.

18. Verfahren nach Anspruch 17, wobei das *wprA*-Gen hinsichtlich mindestens eines Teils des *wprA*-Gens deletiert ist, der CWBP52 kodiert.

19. Verfahren nach Ansprüchen 6 bis 8, wobei der Bakterienstamm *B.subtilis* ist, der eine Deletion mindestens eines Teils der Sequenz enthält, die in Figur 1 von den Nucleinsäurebasen +247 bis +2835 dargestellt ist.

## Revendications

1. Procédé de production d'un polypeptide natif et/ou hétérologue et/ou recombinant à partir d'une souche microbienne ayant une délétion et/ou insertion et/ou mutation et/ou substitution dans un gène *wprA* ou son promoteur correspondant de telle façon que la production et/ou la fonction du produit du gène codé par le gène *wprA* est affectée d'une manière qui facilite la production dudit peptide comprenant :
i) faire croître ladite souche dans des conditions propices à la production et la sécrétion du(es)dit(s) polypeptide(s) ; et
ii) récupérer le(s)dit(s) polypeptide(s) à partir du milieu de croissance et/ou de ladite souche bactérienne.

2. Procédé selon la revendication 1, dans lequel ladite souche microbienne est une souche Gram positive.

3. Procédé selon les revendications 1 ou 2, dans lequel ladite souche microbienne et du genre *Bacillus.*

4. Procédé selon l'une quelconque des revendications 1-3, dans lequel ladite souche bactérienne est *B. subtilis, B. amyloliquifaciens, B. liqueniformis,* ou *B. stearothermophilus.*

5. Procédé selon l'une quelconque des revendications 1-4, dans lequel ledit polypeptide est une hydrolase de carbonyle, carbohydrase, protéase, lipase, amylase, cellulase, oxydoréductase, glycoamylase ou estérase.

6. Procédé selon l'une quelconque des revendications 1-5, dans lequel un gène *wprA* a été altéré par délétion et/ou insertion et/ou mutation et/ou substitution pour empêcher l'expression dudit gène ou pour empêcher la synthèse d'au moins une partie du produit protéique fonctionnel.

7. Procédé selon la revendication 6, dans lequel ledit gène *wprA* est muté par au moins une mutation ponctuelle sélectionnée.

8. Procédé selon les revendications 6 ou 7, dans lequel ledit gène *wprA* a été altéré dans une séquence de contrôle d'expression.

9. Procédé selon l'une quelconque des revendications 6-8, dans lequel ledit gène *wprA* a été altéré par insertion de matériel génétique dans ledit gène *wprA.*

10. Procédé selon la revendication 8, dans lequel ladite séquence de contrôle d'expression est un promoteur.

11. Procédé selon la revendication 10, dans lequel ladite altération résulte en la fourniture d'un promoteur inductible pour contrôler sélectivement l'expression du gène *wprA.*

12. Procédé selon l'une quelconque des revendications 6-8, dans lequel ladite souche bactérienne est *B. subtilis,* contenant une délétion d'au moins une partie de la séquence présentée dans la Figure 1 de la base d'acide nucléique + 154 à +247.

13. Procédé selon la revendication 12, dans lequel ladite souche *B. subtilis* est délétée d'une partie du gène *wprA* codant la séquence signal de la protéine précurseur de WprA.

14. Procédé selon l'une quelconque des revendications 6-8, dans lequel ladite souche bactérienne est *B. subtilis,* contenant une délétion d'au moins une partie de la séquence représentée dans la Figure 1 de la base d'acide nucléique + 154 à +1392.

15. Procédé selon l'une quelconque des revendications 6-8 ou 13, dans lequel ladite délétion comprend au moins une partie de la séquence représentée dans la Figure 1 de la base d'acide nucléique +247 à +1392.

16. Procédé selon la revendication 14, dans lequel le gène *wprA* est délété d'au moins une partie du gène *wprA* codant CWBP23.

17. Procédé selon l'une quelconque des revendications 6-8, dans lequel ladite souche bactérienne est *B. subtilis,* contenant une délétion d'au moins une partie de la séquence représentée dans la Figure 1 de la base d'acide nucléique +1392 à +2835.

18. Procédé selon la revendication 17, dans lequel le gène *wprA* est délété d'au moins une partie du gène *wprA* codant CWBP52.

19. Procédé selon l'une quelconque des revendications 6-8, dans lequel ladite souche bactérienne est *B. subtilis,* contenant une délétion d'au moins une partie de la séquence représentée dans la Figure 1 de la base d'acide nucléique +247 à + 2835.
